(19) Europäisches Patentamt
European Patent Office
Office européen des brevets

(11) Publication number: **0 022 478**
**B1**

(12) **EUROPEAN PATENT SPECIFICATION**

(45) Date of publication of patent specification: **23.02.83**

(21) Application number: **80103286.3**

(22) Date of filing: **12.06.80**

(51) Int. Cl.³: **C 07 D 309/30,**
C 07 C 69/28,
C 07 C 69/30, C 12 P 7/42,
C 12 P 7/62, A 61 K 31/22,
A 61 K 31/365

(54) **Polyhydro-3,7-dimethyl-8-(2-(tetrahydro-4-hydroxy-6-oxo-2H-pyran-2-yl)-ethyl)-1-naphthylenyl-2-methylbutanoates, corresponding hydroxy acids, process for preparing and pharmaceutical compositions containing the same.**

(30) Priority: **15.06.79 US 48946**
**23.01.80 US 114459**
**21.09.79 US 77807**

(43) Date of publication of application:
**21.01.81 Bulletin 81/3**

(45) Publication of the grant of the patent:
**23.02.83 Bulletin 83/8**

(84) Designated Contracting States:
**AT BE CH DE FR GB IT LI LU NL SE**

(56) References cited:
**EP - A - 0 010 951**
**US - A - 4 049 495**

**JOURNAL OF THE CHEMICAL SOCIETE/PERKIN TRANSACTIONS I, No. 11, 1976 A. G. BROWN et al. "Crystal and Molecular Structure of Compactin, a New Antifungal Metabolite from Penicillium Brevicompactum" pages 1165 to 1170**

**Patents Abstracts of Japan, Vol. 4, No. 34, 22 March 1980 page 111C3**

(73) Proprietor: **MERCK & CO. INC.**
**126, East Lincoln Avenue P.O. Box 2000**
**Rahway New Jersey 07065 (US)**

(72) Inventor: **Monaghan, Richard L.**
**48 Johnson Road**
**Somerset New Jersey 08873 (US)**
Inventor: **Alberts, Alfred W.**
**24 Martindale Road**
**Short Hills New Jersey 07078 (US)**
Inventor: **Hoffman, Carl H.**
**1570 Martine Avenue**
**Scotch Plains New Jersey 07076 (US)**
Inventor: **Albers-Schonberg, George**
**30 Tyson Lane**
**Princeton New Jersey 08540 (US)**
Inventor: **Joshua, Henry**
**256 Woodward Avenue**
**Staten Island New York 10314 (US)**
Inventor: **Lopez Aguirre, Maria B.**
**214 Springfield Road**
**Elizabeth New Jersey 07208 (US)**

(74) Representative: **Abitz, Walter, Dr.-Ing. et al,**
**Abitz, Morf, Gritschneder P.O. Box 86 01 09**
**D-8000 München 86 (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

**EP 0 022 478 B1**

Courier Press, Leamington Spa, England

# Polyhydro-3,7-dimethyl-8-[2-(tetrahydro-4-hydroxy-6-oxo-2H-pyran-2-yl)-ethyl]-1-naphthylenyl-2-methylbutanoates, corresponding hydroxy acids, process for preparing and pharmaceutical compositions containing the same

## SUMMARY OF THE INVENTION

This invention relates to hypocholesteremic products from the cultivation of a microfungus of the species *Aspergillus terreus.* More specifically, it relates to compounds of the formulae:

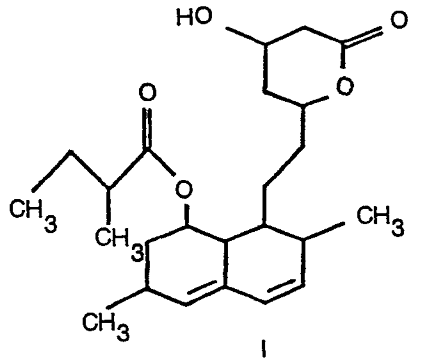

I

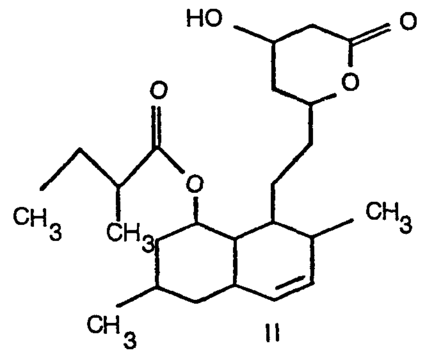

II

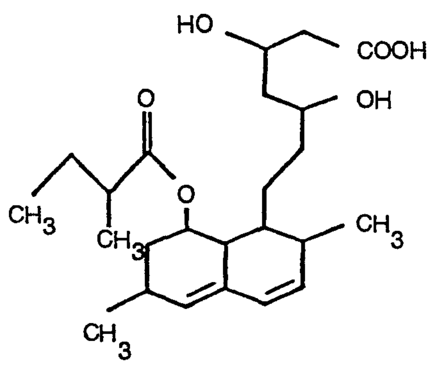

III

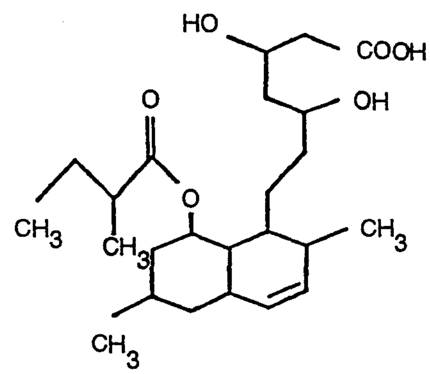

IV

as well as pharmaceutically acceptable salts and lower alkyl and substituted alkyl esters of the carboxylic acids in which the possible substituent is phenyl, dimethylamino or acetylamino. The invention also relates to a process of cultivating the microfungus and isolating from the medium a hypocholesteremic compound of the above structures. These new compounds have excellent properties of inhibiting cholesterol biosynthesis and are useful against hypercholesteremia and hyperlipemia.

## BACKGROUND OF THE INVENTION

Because of the possible connection between high blood cholesterol and atherosclerosis, many efforts have been made to find ways and substances which would reduce the cholesterol in the mammalian body. One of these ways is to inhibit in mammals the body's ability to synthesize cholesterol.

Recently, Endo et al., described (U.S. 4,049,495 and 3,983,140) a fermentation product obtained by cultivation of a microorganism of the genus *Penicillium* and isolation from the medium. They called it ML 236 B and determined its structure together with two related compounds 236 A and 236 C. Its structure, under the name compactin, was also determined by A. G. Brown, T. C. Smale, T. J. King, *J. Chem. Soc.* (Perkin I) 1165 (1975). This compound has been found to be an inhibitor, *in vivo,* of the biosynthesis of cholesterol.

## DESCRIPTION OF THE INVENTION

We have found that unexpectedly, the cultivation of a microorganism very different from that employed by Endo, a microfungus of the species *Aspergillus terreus,* produces new substances that are also very potent inhibitors of the biosynthesis of cholesterol in mammals. We have further found that these substances comprise principally the new compounds I, II, III and IV, of the above structures, accompanied by only traces of other compounds. These new compounds are much more potent inhibitors of cholesterol synthesis *in vivo* that is the compound, ML236B described by Endo.

The pharmaceutically acceptable salts of this invention include those formed from cations such as

sodium, potassium, aluminum, calcium, lithium, magnesium, zinc, ammonia, ethylenediamine, N-methylglucamine, lysine, arginine, ornithine, choline, N,N'-dibenzylethylenediamine, chloroprocaine, diethanolamine, procaine, N-benzylphenethylamine 1-p-chlorobenzyl-2-pyrrolidine-1'-yl-methylbenzimidazole, diethylamine, piperazine, tris-(hydroxymethyl)aminomethane, and tetramethylammonium.

The compounds of this invention are highly useful as antihypercholesteremic agents for the treatment of atherosclerosis, hyperlipemia and like diseases in humans. They many be administered orally or parenterally in the form of a capsule, a tablet, an injectable preparation or the like. It is usually desirable to use the oral route. Doses may be varied, depending on the age, severity, body weight and other conditions of human patients but daily dosage for adults is within a range of from about 2 mg. to 2000 mg. (preferably 2 to 100 mg) which may be given in two to four divided doses. Higher doses may be favourably employed as required.

The compounds of this invention also have useful antifungal activities. For example, they may be used to control strains of *Penicillium sp., Aspergillus niger, Cladosporium sp., Cochliobolus miyabeorus* and *Helminthosporium cynodnotis*. For those utilities they are admixed with suitable formulating agents, powders, emulsifying agents or solvents such as aqueous ethanol and sprayed or dusted on the plants to be protected.

In another aspect of this invention, it relates to a process for producing the compounds of this invention which comprises cultivating a microorganism belonging to the species Aspergillus terreus and then recovering said compounds of this invention from the cultured broth. Based upon taxonomic studies, this Aspergillus, isolated and identified as a hitherto undescribed microorganism, has been designated MF—4833 in the culture collection of Merck and Co., Inc., Rahway, N.J. and a culture thereof has been placed on permanent deposit with the American Type Culture Collection, 12301 Parklawn Drive, Rockville, Maryland 20852, and has been assigned accession number ATCC 20541. Another sample, of a similar organism, designated MR—4845 in the Merck culture collection, has likewise been placed on deposit and has been given the accession number ATCC 20542. The latter organism is the one giving the better yield. Although the use of these is described in connection with the process of this invention, other organisms of the species *Aspergillus terreus* including mutants of the above ones are also capable of producing these novel compounds and their use is contemplated in carrying out the process of this invention.

The morphological characteristics of the microorganisms MF—4833 and MF—4845 have been found to be those of the genus *Aspergillus*. Using the criteria specified in the standard authority "Manual of the Aspergilli", Charles Thom and Kenneth B. Rasper, published by the Williams and Wilkins Company, Baltimore, Md., 1945, and by comparison with known species, it has been determined that both strains are *Aspergillus terreus.* Outstanding characteristics of both cultures of Aspergillus terreus are as follows: They have conidial heads which are compact, columnar and tan deepening to brown as culture ages. The conidiophores are smooth and colorless. The vesicles are hemispherical with upper half to two-thirds covered by sterigmata. Sterigmata occur in two series. The conidia are globose to slightly elliptical and smooth.

The culture of these organisms to produce the novel compounds is carried out in aqueous media such as those employed for the production of other fermentation products. Such media contain sources of carbon, nitrogen and inorganic salts assimilable by the microorganism.

In general, carbohydrates such as sugars, for example, glucose, fructose, maltose, sucrose, xylose, mannitol and the like and starches such as grains, for example oats, ryes, cornstarch, corn meal and the like can be used either alone or in combination as sources of assimilable carbon in the nutrient medium. The exact quantity of the carbohydrate source or sources utilized in the medium depend in part upon the other ingredients of the medium but, in general, the amount of carbohydrate usually varies between about 1% and 6% by weight of the medium. These carbon sources can be used individually, or several such carbon sources may be combined in the medium. In general, many proteinaceous materials may be used as nitrogen sources in the fermentation process. Suitable nitrogen sources include for example, yeast hydrolysates, primary yeast, soybean meal, cottonseed flour, hydrolysates of casein, corn steep liquor, distiller's solubles or tomato paste and the like. The sources of nitrogen either alone or in combination, are used in amounts ranging from about 0.2% to 6% by weight of the aqueous medium.

Among the nutrient inorganic salts which can be incorporated in the culture media are the customary salts capable of yielding sodium, potassium, ammonium, calcium, phosphate, sulfate, chloride, carbonate, and like ions. Also included are trace metals such as cobalt, manganese, iron and magnesium.

It should be noted that the media described in the Examples are merely illustrative of the wide variety of media which may be employed, and are not intended to be limitative. Specifically, the carbon sources used in the culture media to produce the novel compounds of this invention included dextrose, dextrin, oat flour, oatmeal, molasses, citrate, soybean oil, glycerol, malt extract, cod liver oil, starch, ethanol, figs, sodium ascorbate and lard oil. Included as nitrogen sources were peptonized milk, autolyzed yeast, yeast RNA, tomato paste, casein, primary yeast, peanut meal, distillers solubles, corn steep liquor, soybean meal, corn meal, NZ amine, beef extract, asparagine, cottonseed meal and ammonium sulfate. The major ionic components were $CaCo_3$, $KH_2PO_4$, $MgSO_4{\cdot}7H_2O$ and NaCl and small amounts of $CaCl_2{\cdot}6H_2O$ and traces of Fe, Mn, Mo, B and Cu were also present.

The fermentation is carried out at temperatures ranging from about 20° to 37°C; however, for optimum results it is preferable to conduct the fermentation at temperatures of from about 22° to 30°C. The pH of the nutrient media suitable for growing the *Apergillus* culture and producing the novel compounds can vary from about 6.0 to 8.0.

Although the novel compounds are produced by both surface and submerged culture, it is preferred to carry out the fermentation in the submerged state. A small scale fermentation is conveniently carried out by inoculating a suitable nutrient medium with the *Aspergillus* culture and, after transfer to a production medium, permitting the fermentation to proceed at a constant temperature of about 28°C on a shaker for several days.

The fermentation is initiated in a sterilized flask of medium via one or more stages of seed development. The nutrient medium for the seed stage may be any suitable combination of carbon and nitrogen sources. The seed flask is shaken in a constant temperature chamber at about 28°C for 2 days, or until growth is satisfactory, and some of the resulting growth is used to inoculate either a second stage seed or the production medium. Intermediate stage seed flasks, when used, are developed in essentially the same manner, that is, part of the contents of the flask from the last seed stage are used to inoculate the production medium. The inoculated flasks are shaken at a constant temperature for several days, and at the end of the incubation period the contents of the flasks are centrifuged or filtered.

For large scale work, it is preferable to conduct the fermentation in suitable tanks provided with an agitator and a means of aerating the fermentation medium. According to this method, the nutrient medium is made up in the tank and sterilized by heating at temperatures of up to about 120°C. Upon cooling, the sterilized medium is inoculated with a previously grown seed of the producing culture, and the fermentation is permitted to proceed for a period of time as, for example, from 3 to 5 days while agitating and/or aerating the nutrient medium and maintaining the temperature at about 28°C. This method of producing the novel compounds is particularly suited for the preparation of large quantities.

The compounds are conveniently isolated from the fermentation broth as lactones I and II. Alternatively they may be isolated as salts of compounds III and IV.

Compounds I and II can be hydrolyzed with bases such as NaOH to yield the salts such as the sodium salts of compounds III and IV. The use of bases with other pharmaceutically acceptable cations affords salts of these cations. Careful acidification of the salts affords the hydroxy acids III and IV. The hydroxy acids III and IV, can be converted to the compounds I and II at acidic pH. Treating compounds I and II, under acidic or basic catalysis with methanol, ethanol, propanol, or butanol or with phenyl, dimethylamino, or acetylamino alkanols yields the corresponding esters of compounds III and IV which also form a part of this invention.

Compounds III and IV, and especially III can be conveniently isolated without need of chromatography, in the form of the ammonium salts. This isolation is convenient and is much more adapted to commercial use than is chromatography. Furthermore, salts of III and IV are much more active than compounds I and II *in vitro* in the inhibition of cholesterol biosynthesis and as antifungal agents. Indeed the hydroxy acids (and their salts) appear to be the active forms. Therefore, these salts are one of the especially preferred dosage forms. Preferred salts, in addition to ammonium, include tetramethylammonium, and salts of ethylenediamine, sodium potassium, calcium, N-methylglucamine, lysine, arginine and ornithine.

The physico-chemical properties of compound I (MSD—803) are summarized as follows:

| | |
|---|---|
| 1. *Melting point* | 170—171° |
| 2. *Molecular Weight* (mass spectrum) | 404 |
| 3. *Formula* (found by mass spectrometry calculated) | $C_{24}H_{36}O_5$<br>404.2555<br>404.2563 |
| 4. *UV Spectrum* (in acetonitrile): | *Maxima*<br>230.5 nm with E% 505.7<br>237.5 nm with E% 576.6<br>246 nm with E% 395.2 |

5. $^{13}C$ *NMR chemical shifts.* The spectrum has been recorded in $CDCl_3$ solution (20.1 mg in 0.35 ml). Chemical shifts are given relative to internal tetramethylsilane at zero ppm; under the experimental conditions the solvent ($CDCl_3$) signal appears centered at 70.0 ppm. In agreement with mass spectral data 24 carbon atoms are observed; their chemical shifts are:
11.5, 13.6, 16.0, 22.6, 24.1, 26.6, 27.2, 30.5, 32.5, 32.8, 35.9, 36.4, 37.1, 38.4, 41.3, 62.4, 67.8, 76.4, 128.4, 129.7, 131.7, 133.2, 170.8 and 177.2 ppm.

6. *¹H NMR Spectrum*
The spectrum was recorded in $CDCl_3$ solution and chemical shifts are shown in Figure 1 in ppm relative to internal tetramethylsilane at zero ppm.

7. *IR Spectrum*
The infra red spectrum was recorded in a KBr pellet preparation of a sample. It is shown in Figure 2.

8. *Optical rotation*
The specific optical rotation $[\alpha]_D 25 = 320.7°$ has been determined on a solution of 5.30 mg/ml $CH_3CN$. This value has been obtained by measuring at the sodium-D-line wave length.

On the basis of these and other data, the structure of the product is believed, with a considerable degree of certainty, to have the stereo chemical structure:

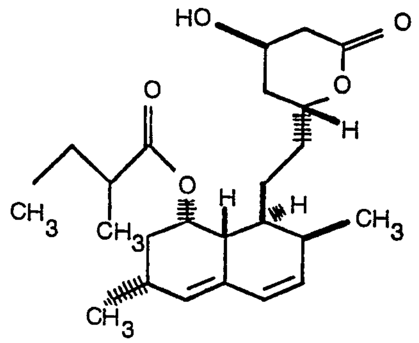

The corresponding hydroxy acid compound III, has the structure:

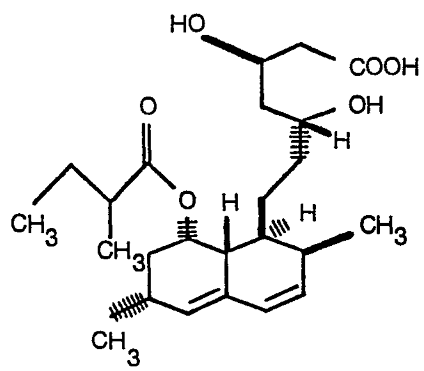

The absolute configuration of the centers of assymetry in these molecules has been determined from X-ray diffraction patterns.

The physico-chemical properties of compound II (MSD—883) are summarized as follows:

1. *Melting Point* 129—131°C

2. *Molecular Weight* 406

3. *Formula* $C_{24}H_{38}O_5$
(found by mass spectrometry 406.2706
calculated) 406.2719

4. *¹H NMR Spectrum*
The spectrum was recorded in $CDCl_3$ solution and chemical shifts are shown in Figure 3 in ppm relative to internal tetramethylsilane at zero ppm.

5. *IR Spectrum*
The infra red spectrum was recorded in a KBr pellet preparation of a sample. It is shown in Figure 4.

6. *Optical rotation*
The specific optical rotation $[\alpha]_D 25 = 148.6°$ has been determined on a solution of 5.23 mg/ml, $CH_3CN$. This value has been obtained by measuring at the sodium-D-line wave length.

7. $^{13}C$ *Nmr Chemical Shifts in $CDCl_3$*
11.8, 14.9, 16.5, 21.1, 23.1, 26.7(2C), 30.9, 31.3, 33.0, 35.7, 35.9, 37.4, 38.5, 38.6, 41.9(2C), 62.3, 70.1, 76.5, 131.0, 132.6, 171.2, 176.7.

On the basis of these and other data, the structure of the product is believed, with a considerable degree of certainty, to have the chemical structure:

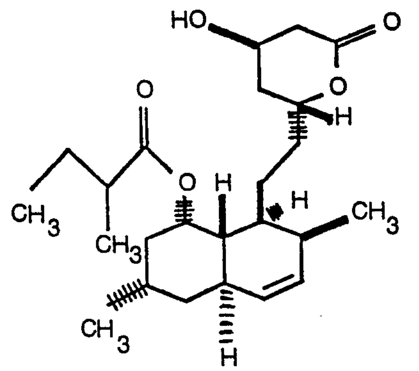

The corresponding hydroxy acid, compound IV, then, has the structure:

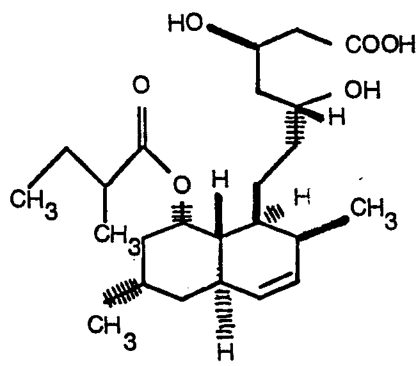

This invention can be illustrated by the following examples.

Example 1
*Preparation of Compounds I and III*

A. Fermentation

A tube of lyophilized culture MF—4833 is opened aseptically and the contents suspended in an unbaffled 250 ml Erlenmeyer flask (seed flask) containing approximately 20 ml of medium A. Medium A has the following composition:

*Medium A*

| | |
|---|---|
| Corn steep liquor | 10 g |
| Tomato paste | 80 g |
| Oatmeal | 20 g |
| Glucose | 20 g |
| Trace Element Mix No. 2 | 20 g |
| Distilled water | 1000 ml |
| pH 6.8 with NaOH | |

*Trace Element Mix No. 2*

| | |
|---|---|
| $FeSO_4 \cdot 7H_2O$ | 1000 mg |
| $MnSO_4 \cdot 4H_2O$ | 1000 mg |
| $CuCl_2 \cdot 2H_2O$ | 25 mg |
| $CaCl_2 \cdot 2H_2O$ | 100 mg |
| $H_3BO_3$ | 56 mg |
| $(NH_4)_6Mo_7O_{24} \cdot 4H_2O$ | 19 mg |
| $ZnSO_4 \cdot 7H_2O$ | 200 mg |
| Distilled Deionized Water | 1000 ml |

The inoculated flask is incubated for 48 hours at 28°C on a 220 rpm shaker (2 inch throw). Two unbaffled 2 liter Erlenmeyer flasks each containing 500 ml of medium B are then each inoculated with 10 ml per flask of the growth from the seed flask. Medium B has the following composition:

*Medium B*

| | |
|---|---|
| Tomato paste | 20 g |
| Primary yeast | 10 g |
| CPC Starch | 20 g |
| $CoCl_2 \cdot 6H_2O$ | 5 mg |
| Distilled water<br>pH 7.2—7.4 with NaOH | 1000 ml |

These two inoculated flasks are incubated for 96 hours at 28°. One flask is incubated without agitation. The other flask is incubated on a 150 rpm shaker (2″ throw). After 96 hours, the contents of each flask is set aside for isolation of the product.

B. Isolation of Compound I

The whole broth is centrifuged for 20—30 min. Solids are saved for extraction. The supernatant liquid (pH 6—8) is charged to a 950 ml bottle and 150 ml XAD-2 resin is added. Using an automatic Extractor, operating on a preset schedule, the mixture is stirred for 2 hours. The spent broth is then siphoned off and discarded. The resin is washed twice with 200 ml of deionized water and the washes were discarded. There then is added a charge of 300 ml of mixed solvent: isopropanol-ethyl acetate-dichloromethane 25—45—30. The mixture is stirred two hours. The solvent-resin slurry is filtered on a Buchner or sintered glass funnel and the resin is discarded. The broth solids are stirred with 100 ml acetone for 1/2 hour. The mixture is then centrifuged and the supernatant liquor is decanted. The combined filtrate and decantate are concentrated to 15 ml.

C. Testing of Compound I

The filtrates were tested as inhibitors of HMG—CoA reductase enzyme by the method described by Beg, Stonik, and Brewer (1977 *FEBS Letters 80* 123 to 129) using enzymes prepared as described by Kleinsek, Rangatham and Porter (1977 *Proc. Nat. Acad. Sci. 74* 1431 to 1435). The positive test (over 90% inhibition at 20 micrograms per milliliter—an $IC_{50}$ of 2.3 micrograms per milliliter) indicated the presence of a very potent inhibitor of sterol synthesis acting at the HMG-CoA reductase level.

Example 2

*Preparation of Compounds I and III*

A. Fermentation

A tube of lyophilized culture of an *Aspergillus* sp. MF—4833 is opened aseptically and the contents suspended in an unbaffled 250 ml Erlenmeyer flask (seed flask No. 1) containing 40 ml of medium C. Medium C has the following compositions:

*Medium C*

| | |
|---|---|
| Corn steep liquor | 5 g |
| Tomato paste | 40 g |
| Oatmeal | 10 g |
| Glucose | 10 g |
| Trace element | 10 g |
| Mix No. 2 | |
| Distilled water<br>pH 6.8 with NaOH | 1000 ml |

This inoculated flask is incubated for 24 hours at 28°C. on a 220 rpm shaker (2 inch throw). Eight more unbaffled 250 ml Erlenmeyer flasks (No. 2 seed flask) each containing 40 ml of medium C are then each inoculated with 2 ml per flask of the growth from seed flask No. 1. These eight No. 2 seed flasks are incubated for 24 hours at 28°C on a 220 rpm shaker (2 inch throw). Twenty, two liter unbaffled Erlenmeyer flasks, containing 500 ml of medium B are then each inoculated with 14 ml per flask of the combined growth of the eight No. 2 seed flasks. These twenty flasks are incubated at 28°, without agitation for 11 days. After 11 days incubation, the contents of these twenty flasks are pooled.

B. Extraction

10.2 liters of whole broth, pH 6.0, was blended in a Waring blender to break up the heavy mycelial pads, centrifuged and the clear supernatant decanted. After filtration the 10 liters of filtrate was extracted with 3 liters of ethyl acetate, yielding 1820 ml of clear extract. A second extraction with 3 liters of ethyl acetate yielded 3350 ml of clear extract. The broth solids were extracted by stirring one hour with 2 liters of methanol and filtering to yield 2100 ml of filtrate.

Aliquots of these extracts were dried and sent for assay by the procedure of Example 1(C), with the following results:

| Extract | | |
|---|---|---|
| Volume (ml) | Total Solids (mg) | Total Units of Activity |
| 1820 | 1133 | 1,496,695 |
| 3350 | 787 | 314,900 |
| 2100 | 13.15 | 1,144,067 |

C. Gel Filtration

The total solids obtained from the first two extracts in Example 2 (B) were combined, dissolved in methanol and filtered to remove insoluble solids. The 30 ml of filtrate was loaded onto a gel filtration column (2.5 cm × 200 cm, 980 ml) packed with Sephadex LH—20 and the sample fractionated according to molecular size using methanol as solvent. With refractive index and U.V. recordings as guides, the best fractions were identified by Bioassay.

| Total Solids (mg) | Total Units of Activity |
|---|---|
| Fraction 1 — 89 | 106,271 |
| Fraction 2 — 278 | 1,099,680 |
| Fraction 3 — 779 | 210,357 |

D. Separation and Purification

A sample from Fraction 2 above was prefiltered through a 1-gram bed of Waters Bondapak C18/Porasil B and eluted with five volumes of methanol. The methanol eluate was concentrated to 0.5 ml. This sample was chromatographed, over several runs, on a Waters $\mu$C18 column (3.9 mm × 30 cm) with methanol: 0.05 *M* ammonium phosphate, pH 2.9 (75:25), as the developing solvent. Fractions were scanned on a Beckman Spectrophotometer, and those showing absorption maxima at 236 nm,

with shoulders at 229 nm and 245 nm were combined and concentrated under reduced pressure to an aqueous solution. The pH of the concentrate was adjusted to 6.5 with 2 *M* potassium hydroxide and the active components were extracted with ethyl acetate. The organic layer was dried, concentrated to dryness, and the residue was dissolved in 0.3 ml methanol. The methanol solution was chromatographed as above and recycled. Cuts containing earlier eluting component were combined, concentrated to an aqueous solution and extracted with chloroform. The chloroform residue was taken up in methanol and the solvent evaporated under nitrogen. 3.5 mg. of dried product was obtained and identified as hydroxy acid (compound III). Cuts containing the second component were combined and extracted with chloroform as above. 0.87 Mg of dried product was obtained and identified as lactone, (compound I).

Example 3

*Best Mode of Fermentation of MF—4833 to Produce Compounds I and III*

A tube of lyophilized culture of an *Aspergillus* sp. MF—4833 is opened aseptically and the contents suspended in an unbaffled 250 ml Erlenmeyer flask (seed flask) containing 40 ml of medium C. The inoculated flask is incubated for 48 hours at 28°C on a 220 rpm shaker (2 inch throw). Two, 250 ml unbaffled Erlenmeyer flasks each containing 40 ml of medium D are then each inoculated with 2 ml per flask of the growth from the seed flask. Medium D has the following composition:

*Medium D*

| | |
|---|---|
| Lactose | 20 g |
| Distillers solubles | 15 g |
| Autolyzed yeast | 5 g |
| Distilled water<br>ph 7.0 with NaOH | 1000 ml |

These two inoculated flasks are incubated for 96 hours at 28° on a 150 rpm shaker (2 inch throw). After 96 hours incubation the contents of these two flasks is submitted for extraction by the procedure described in Example 2(B). Total production in these flasks is 1450—2000 units/ml.

Example 4

*Preparation of Compounds I and III*

A tube of lyophilized culture of an *Aspergillus*, MF 4845, is opened aseptically and the contents suspended in an unbaffled 250 ml Erlenmeyer flask (seed flask No. 1) containing 40 ml of medium C. The inoculated flask is incubated for 24—48 hours at 28°C on a 220 rpm shaker (2 inch throw). A portion (approx. 0.5 ml) of this flask is then used to inoculate a slant tube containing medium E. Medium E has the following composition:

*Medium E*

| | |
|---|---|
| Yeast Extract | 4 g |
| Malt Extract | 10 g |
| Dextrose | 4 g |
| Agar | 20 g |
| Distilled water<br>pH 7.0 with NaOH | 1000 ml |

The inoculated slant tube is incubated for 11 days at room temperature. It is then stored at −60°C for 3—4 months. A portion of the contents of this slant is then suspended in an unbaffled, 250 ml Erlenmeyer flask (No. 2 seed flask) containing 40 ml of medium C. The inoculated flask is incubated for 24 hours at 28°C on a 220 rpm shaker (2 inch throw). Six unbaffled 250 ml Erlenmeyer flasks (No. 3 seed flasks) containing 40 ml of medium C are then each inoculated with 2 ml per flask of the growth from the No. 2 seed flask. These six inoculated flasks are incubated for 48 hours at 28° on a 220 rpm shaker (2 inch throw). Six unbaffled two liter Erlenmeyer flasks containing 500 ml of medium F are each then inoculated with the contents of No. 3 seed flask. Medium F has the following composition:

*Medium F*

| | |
|---|---|
| Corn steep liquor | 15 g |
| CPC Starch | 20 g |
| Corn meal | 1 g |
| Soybean meal | 4 g |
| Glucose | 5 g |
| Soybean oil | 2.5 g |
| $(NH_4)_2SO_4$ | 4 g |
| $KH_2PO_4$ | 0.3 g |
| $CaCO_3$ | 6 g |
| Distilled water<br>pH 6.7 with NaOH | 1000 ml |

The inoculated flasks are incubated for 11 days without agitation at 28°C. After 11 days broth is delivered for extraction by the procedure of Example 2(B). Total production in these flasks is 1231 units/ml.

Example 5

*Best Mode of Fermentation with MF—4845 to Produce Compounds I and III*

A tube of lyophilized culture of an *Aspergillus,* MF—4845, is opened aseptically and the contents suspended in an unbaffled 250 ml Erlenmeyer flask (seed flask) containing 40 ml of medium C. The inoculated flask is incubated for 30 hours at 28° on a 220 rpm shaker (2 inch throw). An unbaffled, 250 ml Erlenmeyer flask containing 40 ml of medium G is inoculated with 2 ml per flask of the growth from the seed flask. Medium G has the following composition:

*Medium G*

| | |
|---|---|
| Dextrose | 45 g |
| Peptonized milk | 24 g |
| Autolyzed yeast | 2.5 g |
| Polyglycol P2000 | 2.5 ml |
| Distilled water<br>pH 7.0 with NaOH | 1000 ml |

This inoculated flask is incubated for 120 hours at 28°C on a 220 rpm shaker (2 inch throw). After 120 hours incubation, the contents of the flask is submitted for extraction by the procedure of Example 2 (B). Total production in this flask is 21,500 units/ml.

Example 6

A. Large Scale Fermentation with MF—4833 to Produce Compounds I and III

The medium used in each step of the fermentation comprised:

| | |
|---|---|
| Corn steep liquor | 5 g |
| Tomato paste | 40 g |
| Oat Flour | 10 g |
| Glucose | 10 g |
| Trace element solution | 10 ml |
| Distilled water | 1000 ml |

adjusted to pH 6.8 with sodium hydroxide.

The trace element solution comprised:

| | |
|---|---|
| $FeSO_4 \cdot 7H_2O$ | 1 g |
| $MnSO_4 \cdot 4H_2O$ | 1 g |
| $CuCl_2 \cdot 2H_2O$ | 25 mg |
| $CaCl_2$ | 100 mg |
| $H_3BO_3$ | 56 mg |
| $(NH_4)_6 Mo_7O_{24} \cdot 4H_2O$ | 19 mg |
| $Zn SO_4 \cdot 7H_2O$ | 200 mg |
| distilled water | 1 liter |

All media were checked for sterility before inoculation with a microorganism.

To a 250 ml non-baffled Erlenmeyer flask was charged 40 ml of medium and the contents of one tube of lyophillized organism MF 4833. It was then shaken for 24 hours at 28°C on a rotary shaker at 220 rpm. New flasks were then charged with 40 ml of medium and 1 ml of the first flask's contents and were shaken an additional 24 hours at 28°C. A 2 liter flask was then charged with 400 ml of medium and 10 ml of the second stage fermentation mixture and this too was shaken for 24 hours at 28°C.

A 200 gallon stainless steel fermentation vat was then charged with 501 liters of a medium comprising:

| | |
|---|---|
| lactose | 2% wt/vol |
| distiller solubles | 1.5% wt/vol |
| autolyzed yeast | 0.5% wt/vol |
| Polyglycol P2000 | 0.25% wt/vol |

whose pH was adjusted to 7.0. This was sterilized 15 minutes at 121°C. One liter of the third stage above was then charged and the mixture was incubated at 130 rpm at 28°C for 96 hours with an air flow of 10 cfm.

B. Isolation of Compound I

About 37.5 lbs. (3/4 bag) of a silicaceous filter aid was added to 110 gal. whole broth from the culture of MF—4833 described above and the mixture was filtered through an 18-inch filter press. The clarified filtrate, (pH 6.6) was adjusted to pH 4.0 by careful addition of 450 ml of concentrated hydrochloric acid, and extracted by agitation with about one-third volume (36 gal.) of ethyl acetate. After separation, the upper solvent layer was removed, and the water phase again extracted with ethyl acetate (38 gal.) in a similar fashion. After separation, the two extracts were combined and backwashed by agitation with about twelve gallons of water. After separation, the ethyl acetate solution was concentrated under vacuum at a temperature below 30°C, first in a stirred kettle, and finally in a rotary vacuum evaporator to a residual volume of slightly less than one gallon.

Approximately 1 gal. (3800 ml) of ethyl acetate concentrate from the preceding extraction was further concentrated in a rotary evaporator (*ca* 10 mm, 40°C bath) to a syrup and was then concentrated twice more, after addition of about one liter of methylene chloride in two portions, to free the syrup of polar solvent. The final oil of about 300 ml which contained about 250 g of solids by dry weight determination, was made up to about 750 ml with ethyl acetate methylene chloride (30/70; v/v) and 200 g of silica gel was added and mixed in to form a slurry. This was layered over the top of a 14 cm by 36 cm column bed holding 2.5 kg of the same silica gel, in about 7.5 1 volume, which had been packed as a slurry in the same solvent mixture. Development with the same solvent was continued until 3 liters of effluent was taken off as forerun.

Development with ethyl acetate-methylene chloride (50/50; v/v) was begun, taking 800 ml effluent fractions. Twelve fractions were taken, then 100% ethyl acetate elution was begun, and after seven more fractions, 100% acetone elution was begun. Fractions four through twenty-four were assayed for bio-activity in the HMG-CoA Reductase inhibition assay referred to in Example 1. Substantial activity was found in fraction 7 through 11. Peak activity was found in fraction 8. It was concentrated to an oil for further purification; dry wt. by solids determination was 9.0 gm.

Fraction 8 from the silica gel column was triturated with 50 ml methylene chloride and filtered;

the dried filter cake weighed 4.9 gm. The filtrate was charged to a 2-inch I.D. by 1-meter long column filled with Sephadex LH—20 dextran gel (Pharmacia) swollen and equilibrated in methylene chloride, and the column was eluted with methylene chloride at a rate of 15 ml/min. Compound I was eluted between 0.64 and 0.81 column volumes. Solvent was removed from this fraction leaving a slightly brown residue weighing approximately 0.290 gm. This residue (213 mg) was taken up in 1.5 ml of $CH_2Cl_2$—$CH_3CN$ (65—35), charged to a prepacked and equilibrated silica gel column (EM LOBAR Size B) and eluted with $CH_2Cl_2$—$CH_3CN$ (65—35) at 5 ml/min. Evaporation of solvent from the peak eluting between 235 and 360 ml of eluant left 121 mg of crystalline product, m.p. 155—160°C. HPLC of this material on a EM RP 18 reverse-phase analytical column (E. Merck HIBAR II, Cat. No. 906046) using 0.05M sodium phosphate pH 3.0-acetonitrile 45—55 as eluant at 2 ml/min. showed a characteristic uv absorbing peak at 11 min.

Eighty-two mg of this material was recrystallized from 0.6 ml of absolute ethanol, then again from 0.4 ml of the same solvent to afford, after drying over-night in a desiccator over $P_2O_5$, 40 mg of white feathery crystals. Analytical HPLC on the system described above gave a single sharp peak at 11 minutes elution time. After further recrystallizations, a melting point of 170—171°C was obtained.

The product was identified by spectra, etc., as compound I. This material, in the in vitro HMG-CoA reductase test (of Example 1) gave an $IC_{50}$ of 0.01 micrograms per milliliter.

## Example 7

*Direct Isolation of the Ammonium Salt of Compound III*

The broth from a fermentation as in Example 20A (100 gal) is acidified with $H_3PO_4$ to pH of 5. Ethyl acetate (70 gal) is added and the mixture is stirred vigorously. It is then filtered from the mycelia residue and the cake is washed with a small amount of ethyl acetate which is combined with the main extract. The organic phase is separated and mixed with 5 gallons of 0.2N sodium hydroxide solution. The mixture is stirred vigorously and then allowed to settle. The aqueous layer is separated and the pH is adjusted from 9 to 5 by addition of $H_3PO_4$. It is then extracted, first with 2 gallons of hexane-ethyl acetate 2:1 mixture and then with one gallon of the same mixture. The separate organic extracts are combined and dried over anhydrous $MgSO_4$. The drying agent is then separated by filtration and the cake washed with one liter of the same hexane-ethyl acetate solution, the rinse being combined with the filtrate. This filtrate solution, after further dilution with 2 L of acetone, is stirred while ammonia gas is passed in. The gas is absorbed and a crystalline precipitate appears. When ammonia is no longer absorbed, and a darkening in color is observed in the precipitate, the introduction of ammonia is terminated and the mixture is allowed to stand several hours after which it is filtered. The crude ammonium salt filter cake is washed with acetone to a colorless wash and is then air dried.

The crude ammonium salt can be recrystallized by dissolving it in 1.5 gallons of a mixture of chloroform, methanol and concentrated aqueous ammonium hydroxide (80:20:2) and filtering the colored insoluble material. The filtrate solution is then diluted with an equal volume of acetone-ether 1:1 combination and allowed to stand overnight. The crystalline, tan colored ammonium salt (96.5 g) is obtained by filtration.

Further purification can be achieved by dissolving 70 g of the above recrystallized material in 2 liters of boiling isopropanol-concentrated aqueous $NH_4OH$ (95:5). Ten grams of activated charcoal are added and the hot solution is filtered. The filtrate is allowed to cool to room temperature and stored overnight at −20°C. Filtration is followed by washing the cake with cold (−20°) isopropanol followed by cold acetone and then room temperature ether. The product is dried under nitrogen, to give about 60 g of white crystalline ammonium salt.

## Example 8

*Salts of Compound III*

To a solution of 40 mg of the product of Example 6 in 2 ml of ethanol is added 1 ml of aqueous NaOH ($10^{-4}$ moles; 1 equivalent). After one hour at room temperature, the mixture is taken to dryness *in vacuo* to yield the sodium salt of Compound III.

In like manner the potassium salt is prepared using one equivalent of potassium hydroxide.

## Example 9

*L-Lysine Salt of Compound III*

A solution of 146 mg of L-lysine in 1.5 ml of 65% ethanol is added to a solution of 440 mg of the ammonium salt of Compound III in 11.5 ml of 85% ethanol. The solvents are distilled off *in vacuo*. The residue is triturated with 10 ml of warm ethanol, cooled, and filtered, and the white solid is dried to obtain 430 mg of the L-lysine salt of Compound III, m.p. 178—180° (d).

| | |
|---|---|
| Anal. Calcd. for $C_{30}H_{52}N_2O_8$: | C, 63.35; H, 9.22, N, 4.93; |
| Found: | C, 62.80; H, 9.13; N, 4.83. |

Example 10

*L-Arginine Salt of Compound III*

In the manner substantially as described in Example 9, a solution of 174 mg of L-arginine base and a solution of 440 mg of the ammonium salt of Compound III are combined. The solvent is evaporated *in vacuo*, and the residue is triturated with warm ethanol, cooled, filtered, and dried to give the L-arginine salt of Compound III.

Example 11

*L-Ornithine Salt of Compound III*

In the manner substantially as described in Example 9, a solution of 132 mg of L-ornithine free base and a solution of 440 mg of ammonium salt of Compound III are combined. The solvent is evaporated *in vacuo* and the residue is triturated with warm ethanol, cooled, filtered, and dried to give the L-ornithine salt of Compound III.

Example 12

*N-Methylglucamine Salt of Compound III*

In the manner substantially as described in Example 9, a solution of 195 mg of N-methylglucamine in 1.5 ml of water and 440 mg of Compound III ammonium salt in 11.5 ml of 85% ethanol are combined. The solvent is evaporated *in vacuo* to obtain the N-methylglucamine salt of Compound III.

Example 13

*Ethylenediamine Salt of Compound III*

18 g of Compound I are dissolved in 180 ml of warm isopropanol and treated with 90 ml of 0.5M aqueous NaOH solution, aged one hour, diluted with 180 ml of water, evaporated *in vacuo* to remove the isopropanol, and cooled in an ice bath. Slowly, 90 ml of 0.5M HCl is added and the mixture is extracted with 2 x 150 ml of ethyl acetate which is backwashed with 100 ml of water, dried over $MgSO_4$. The solvent is removed *in vacuo* at low temperature and the residue is dissolved in 150 ml of ethanol. 3 Ml of ethylene diamine is added and the solvent is evaporated *in vacuo*, and the residue triturated with boiling ethyl acetate, cooled, filtered, and recrystallized from 30 ml of isopropanol and dried *in vacuo* over $P_2O_5$ to obtain 13.1 g of white crystals, m.p. 152—153.5°C.

| | |
|---|---|
| Anal. Calcd. for $(C_{24}H_{37}O_6)_2^- \cdot (C_2H_{10}N_2)^{++}$: | C, 66.35; H, 9.35; N, 3.09; |
| Found: | C, 66.08; H, 9.49; N, 3.01 |

Example 14

*Calcium Salt of Compound III*

87.9 mg ammonium salt of Compound III are dissolved in 3 ml $H_2O$ with stirring and heating. 7.4 Mg analytical grade $Ca(OH)_2$ are then added and the mixture stirred and heated until no more ammonia evaporates and only a slight turbidity remains which is separated by centrifugation. The colorless, clear supernatant is lyophilized and probes of the dry material set up for crystallization from various solvents and solvent mixtures. The product crystallizes in needles when a hot concentrated solution in dry isopropanol is allowed to cool to room temperature.

Example 15

*Tetramethylammonium Salt of Compound III*

34 Mg Compound I in 1 ml $CH_2Cl_2$ are treated with 0.04 ml 24% tetramethylammonium hydroxide in methanol. The product is precipitated with ether in partially crystalline form, centrifuged and the precipitate first washed with ether and then recrystallized as hexagonal plates from 1 ml isopropanol by the addition of 5 ml ether and about 5 ml low boiling petrol ether. 27 Mg or 65% are obtained.

$^1H$ Nmr Spectrum of Compound III Tetramethylammonium Salt

(6 mg/0.35 ml at 25°C in $CDCl_3$ at 300 MHz)
0.83 t (3*H*, J = 6.5)
0.84 d (3*H*, J = 7)
1.02 d (3*H*, J = 7)
1.05 d (3*H*, J = 7)
1.24 m (~1*H*); 1.30—1.80 br.m. envelope

1.88 ddd (1*H*, J = 2, 8, 15)
1.98 dd (1*H*, 3, 15)
2.16 dd (1*H*, J = 8.5, 15.5)
2.23 m (1*H*, obscured)

2.32 m (1*H*, obscured)
2.37 dd (1*H*, J = 3, 15.5)
2.40 m (~1*H*, obscured)
3.42 s (12*H*, $MeN^+$)
3.79 m (1*H*, symmetrical multiplet)
4.06 m (1*H*, symmetrical multiplet)
5.32 dt (1*H*, J ~ 3)
5.50 br.s (1*H*)
5.79 dd (1*H*, J = 6,10)
5.98 d (1*H*, J = 10)

Chemical shifts are in ppm downfield of internal TMS. Coupling constants in brackets are in Hz. Abbreviations: s = singlet, d = doublet, t = triplet, m = multiplet

Example 16

*Ammonium Salt of Compound III*

In the manner substantially as described in Example 13, Compound I is converted to the hydroxy acid, Compound III, extracted into ethyl acetate, dried over $MgSO_4$, filtered, and treated with anhydrous ammonia with stirring and cooling to precipitate the ammonium salt.

Example 17

*Preparation of Hydroxy acid, Compound III*

The sodium salt produced in Example 7 is redissolved in 2 ml of ethanol-water (1:1) and added to 10 ml of 0.1N hydrochloric acid from which the liberated hydroxy acid is extracted with ethyl acetate. The latter solvent is washed once with water, dried and removed *in vacuo* with a bath temperature not exceeding 30°. The hydroxy acid derived slowly reverts to the lactone on standing.

Example 18

*Compound III*

453 Mg of the ethylenediamine salt of Compound III are dissolved in 6 ml of 80% ethanol, cooled in an ice bath, treated with 1 ml of 1M HCl, evaporated *in vacuo* to remove the ethanol, treated with 3 ml more water, extracted into 2 x 5 ml of ethyl acetate, and backwashed with water, keeping all solutions cold in an ice bath. The extract is dried over $MgSO_4$ and concentrated to dryness *in vacuo* to obtain the hydroxy acid as a colorless oil.

A $^{13}C$-nmr spectrum in $CDCl_3$ (190 mg/ml) exhibits chemical shifts for the first six carbons of the hydroxy acid moiety as listed in the table. Upon standing, this hydroxy acid slowly reverts to the lactone.

TABLE

$^{13}C$-Nmr Spectrum, Ppm Downfield from Tetramethylsilane

| | | Hydroxy Acid, Compound III |
|---|---|---|
| 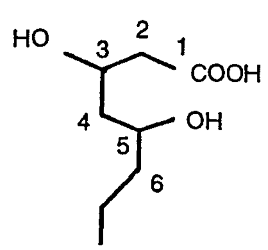 | $C_1$ | 174.8 |
| | $C_2$, $C_4$ | 42.4, 41.6 |
| | $C_3$ | 68.8 |
| | $C_5$ | 72.3 |
| | $C_6$ | 34.9 |

The spectrum of the remainder of the molecule is only slightly changed by the ring closure.

Example 19

*Ethyl Ester of Compound III*

A suspension of 500 mg (1.24 mmol) of Compound I (MSD—803) in 20 ml of ethanol is stirred at room temperature under a nitrogen atmosphere. A small piece of sodium (*Ca*. 1 mg) is added. After 15 minutes a second small piece of sodium is added. After a total reaction time of 30 minutes the homogeneous reaction mixture is diluted with ether, washed with water and with saturated brine and dried ($MgSO_4$). Evaporation of the solvent gives a waxy solid. Analysis by HPLC on a Whatman Partasil 10 PAC column (4.6 mm x 25 cm) with 10% isopropanol/hexane pumped at 6 ml/min indicated a mixture of ethyl ester and MSD—803 (77:23). This mixture is separated by medium-pressure

chromatography on silica gel (230—400 mesh) by elution with 3% ethanol/methylene chloride. The fractions containing the ester are combined and evaporated to give 358 mg (66%) of an off-white solid, m.p. 67°C.

A portion of this material is recrystallized from hexane to give white needles: m.p. 66.5—68.5°.

| | |
|---|---|
| Anal. Calcd. for $C_{26}H_{42}O_6$: | C, 69.30; H, 9.40 |
| Found: | C, 69.22; H, 9.58 |

In like manner, by the use of equivalent amounts of methanol, propanol, butanol, isobutanol, t-butanol, amyl alcohol, isoamyl alcohol, 2-dimethylaminoethanol, benzyl alcohol, phenethanol, 2-acetamidoethanol and the like, the corresponding esters are obtained.

Example 20

*Preparation of Compounds II and IV*

A. Fermentation

A tube of lyophilized culture MF—4845 is opened aseptically and the contents suspended in an unbaffled 250 ml Erlenmeyer flask (seed flask) containing approximately 10 ml of the Medium which has the following composition:

| *Medium* | |
|---|---|
| Corn steep liquor | 5 g |
| Tomato paste | 40 g |
| Oatmeal | 10 g |
| Glucose | 10 g |
| Trace Element Solution | 10 g |
| Distilled water<br>pH 6.8 with NaOH | 1000 ml |
| *Trace Element Solution:* | |
| $FeSO_4 \cdot 7H_2O$ | 1000 mg |
| $MnSO_4 \cdot 4H_2O$ | 1000 mg |
| $CuCl_2 \cdot 2H_2O$ | 25 mg |
| $CaCl_2 \cdot 2H_2O$ | 100 mg |
| $H_3BO_3$ | 56 mg |
| $(NH_4)_6Mo_7O_{24} \cdot 4H_2O$ | 19 mg |
| $ZnSO_4 \cdot 7H_2O$ | 200 mg |
| Distilled Deionized Water | 1000 ml |

The inoculated flask is incubated for 24 hours at 28°C on a 220 rpm shaker (2 inch throw). An unbaffled 2 liter Erlenmeyer flask containing 500 ml of the medium is then inoculated with 10 ml of the first stage fermentation growth from the seed mixture. This too was shaken 24 hours at 28°C.

A 200 gallon stainless steel fermentation vat was then charged with 485 liters of a medium comprising:

| | |
|---|---|
| Cerelose | 4.5% wt/vol |
| Peptonized Milk | 2.5% wt/vol |
| Autolyzed yeast | 0.25% wt/vol |
| Polyglycol P2000 | 0.25% vol/vol |

whose pH was adjusted to 7.0. This was sterilized 15 minutes at 121°C. One liter of the second stage above was then charged and the mixture was incubated at 85 rpm for 12 hours then 130 rpm for 84 hours at 28°C with an air flow of 5 cfm for 12 hours then 10 cfm for 84 hours.

B. Isolation

1. Extraction

Two batches of one hundred gallons of whole broth were combined, acidified with stirring to pH 4.1 by careful addition of 800 ml of concentrated hydrochloric acid, and extracted by addition of 75 gal of ethyl acetate and further stirring for two hours.

About 25 lbs of a silicaceous filter aid was then added and the total slurry was pumped through a 24-inch filter press. An additional 75 gal of ethyl acetate was used to wash the press cake and continue the extraction, by reversing the direction of pumping through the press four times. Then all of the wash solvent was discharged from the press and combined with the first filtrate. The two-phase filtrate was allowed to settle, and the water layer removed. The ethyl acetate layer was washed with 10 gal of deionized water, the phases were allowed to separate and the ethyl acetate extracts were concentrated under vacuum to a residue of about 10 gal.

2. Lactonization

(In order to cyclize any Compound IV in the extract to Compound II, the following azeotropic treatment was performed.)

Ethyl acetate extracts from an additional three hundred gal of broth were added to the above extract and the volume was reduced to about thirty gal by vacuum distillation. About fifty gal of toluene was added, and the batch was concentrated under vacuum to 32 gal; this step was repeated; then sufficient new toluene was added to bring the volume to 75 gal. Without vacuum, the batch was brought to reflux and maintained there for two hours, with a temperature over 106°C.

This solution was then concentrated under vacuum to a small volume, which was further concentrated to an oily residue in a large rotary evaporator under vacuum.

3. Chromatography on Silica Gel

The extract obtained above was flushed free of other solvents by addition of 2 gal of methylene chloride and reconcentration to an oil.

The oily residue was dissolved in about 5 gal of ethyl acetate-methylene chloride (30/70; v/v) mixture, and a slurry was made by addition of 2.8 kg of silica gel.

The slurry was loaded as a level layer on the top of a 12 in. x 50 in. silica gel column packed in the same solvent mixture.

Elution was with ethyl acetate-methylene chloride (40/60; v/v) at 800 ml/min. A forerun of 10 gal, then further fractions of 4 gal each were collected.

Fractions 6—10 inclusive were concentrated under vacuum to an oily residue which was dissolved in hot ethyl acetate, treated with decolorizing carbon, filtered hot, and cooled. Crystals of MSD 803 (Compound I) were filtered off and the mother liquors were concentrated to an oil for further chromatography.

4. Rechromatography on Silica Gel

Mother liquor residues from similar broth extract work-ups equivalent to an additional 600 gal of fermentation production were combined with the above in methylene chloride solution. One-half of this solution was taken for further silica gel chromatography. A small aliquot showed a total solids content of 325 g. The solution was treated with 40 g of decolorizing carbon, filtered, and the cake rinsed with methylene chloride. The combined filtrate and washings were concentrated under vacuum to an oily residue. This was redissolved in 800 ml of ethyl acetate methylene chloride (30/70; v/v) and slurried with 225 g of silica gel. The slurry was loaded on top of a 14 x 36 cm column bed of silica gel packed in the same solvent mixture. Development was with ethyl acetate methylene chloride (40/60; v/v). A forecut of three liters was set aside; then fractions of 800 ml each were collected.

5. Chromatography on Reverse-phase Packing

Forty ml from fraction 12 of the above chromatography were concentrated to an oil weighing 500 mg and the oil redissolved in 5 ml acetonitrile. This acetonitrile solution was charged to a 5/8" OD by 6 ft long stainless steel chromatography column packed with preparative reverse-phase liquid chromatography column packing material "Bondapak C18/PorasilB" (Water Associates, Inc., Milford, Mass. 01757). The column was eluted with a mixture consisting of v/v 55% acetonitrile and 45% 0.05 M ammonium phosphate pH3. The elution volume between 1360 ml and 1700 ml was combined on the basis of refractive index detection. The organic solvent was removed *in vacuo* and the residual aqueous solution extracted with ethyl acetate. *In vacuo* removal of the ethyl acetate left 120 mg of the title compound which crystallized from a concentrated acetonitrile solution yielding crystals MSD 883 (Compound II), m.p. 129—131°C.

Example 21

*Alternate Isolation of Compounds II and IV*

Crude ammonium salt, isolated as described (in Example 7) from a 1100 gal fermentation, is lactonized by dissolving in water, acidifying to pH3 with concentrated hydrochloric acid, extracting into toluene, and refluxing for two hours, as described in Example 20—B—2. Concentration under a vacuum to a small volume affords crystals of crude Compound I mixed with a smaller amount of Compound II; these are filtered off and dried.

Material from twelve such batches is combined and recrystallized from 101 lbs of ethanol, filtered, and washed with ethanol. The combined mother liquors and washes are concentrated *in vacuo* to about 3 gal and a second crop of crystals of Compound I are filtered off. The mother liquors from this filtration are worked up as follows:

About 0.5 l of these mother liquors are prepared for reverse-phase chromatography by concentration under vacuum to remove ethanol, flushing with acetonitrile and filtration of trace insolubles. A solution in 100 ml of acetonitrile is passed over a 200 ml bed of C—18 reverse-phase packing, and the bed washed with an additional liter of acetonitrile. The combined filtrates are concentrated and dissolved in a total of 360 ml of chromatographic solvent (60 acetonitrile — 40 water v/v) and filtered to remove trace insolubles. Solids content by test aliquot is 15.3 g.

A charge of 160 ml of this feed stock is chromatographed in a Waters Prep 500 system using two 5 x 30 cm cartridges of C—18 reverse-phase packing (Waters Assoc.; bonded octa decyl coating on silica) and acetonitrile-water, 60—40 (v/v) as the eluant at 130 ml/min, at ambient temperature, with refractive index detection. Impurities eluted in the first 3900 ml of eluant are rejected. A large peak of Compound I is obtained from 3900 to 5850 ml of eluant and set aside. Mixed fractions are obtained from 5850 ml to 6500 ml of elution; these are set aside for rechromatography. Purified Compound II is obtained as the last peak; the fraction between 6500 and 8450 ml of elution is collected and concentrated to an oily residue of about 3 g.

One-half (about 1.5 g) of this concentrate of Compound II is prepared for chromatography as the ammonium salt of Compound IV by making a solution in 6 ml of warm methanol with agitation and adding immediately sufficient 1*N* sodium hydroxide to hold the pH at 10.5—11; 3.5—3.8 ml of alkali are required. After standing for one-half hour, the solution is filtered to remove a trace of insolubles, and the filtrate is pumped into a column, 1" x 34" of 200—325 mesh milled XAD—2 resin (a styrenedivinylbenzene copolymer) and eluted with 23% acetonitrile —77% 0.1N ammonium hydroxide at 2 ml/min at 40°C. Fractions of 20 ml are collected; fractions 1 through 44 contain impurities, and a peak of Compound III salt, which is rejected. Fractions 45—61 are collected, concentrated under vacuum to a small aqueous residual volume and freeze dried to a residue of ammonium salt of Compound IV, wt. 0.72 g.

Example 22

*Salts of Compound IV*

To a solution of 40 mg of the product of Example 20 in 2 ml of ethanol is added 1 ml of aqueous NaOH ($10^{-4}$ moles; 1 equivalent). After one hour at room temperature, the mixture is taken to dryness *in vacuo* to yield the sodium salt of Compound IV.

In like manner the potassium salt is prepared using one equivalent of potassium hydroxide and the calcium salt using one-half equivalent of CaO.

Employing the procedures substantially as described in Examples 9—12 and 14, but substituting for the ammonium salt of Compound III in each case an equivalent amount of the ammonium salt of Compound IV, there is produced respectively the L-lysine, L-arginine, L-ornithine, N-methylglucamine and calcium salts of compound IV.

Employing the procedures substantially as described in Examples 13, 15 and 16, but substituting for Compound I in each case, an equivalent amount of Compound II, there is produced respectively the ethylene diamine, tetramethylammonium and ammonium salts of Compound IV.

Example 23

*Preparation of Hydroxy Acid, Compound IV*

The sodium salt produced in Example 22 is redissolved in 2 ml of ethanol-water (1:1) and added to 10 ml of 0.1N hydrochloric acid from which the liberated hydroxy acid is extracted with ethyl acetate. The latter solvent is washed once with water, dried and removed *in vacuo* with a bath temperature not exceeding 30°. The hydroxy acid derived slowly reverts to the lactone on standing.

Example 24

*Preparation of Hydroxy Acid, Compound IV*

221 Mg of the ammonium salt of Compound IV, are dissolved in 4.5 ml of 65% ethanol. Cooled in ice, acidified with about 0.5 ml of 1M HCl to pH3, and evaporated at low temperature in a rotary evaporator to a volume of about 2 ml. 2 Ml more water are added and extracted into 2 x 3 ml of ethyl acetate, and backwashed with 1 ml of water, keeping all solutions cold in an ice bath. The extract is dried over $MgSO_4$ and evaporated to dryness *in vacuo* to obtain the hydroxy acid as a colorless oil.

A $^{13}C$-nmr spectrum in $CDCl_3$ exhibits chemical shifts for the first six carbons of the hydroxy acid part of the molecule as listed in the Table. Upon standing, this hydroxy acid slowly reverts to the lactone.

Table

$^{13}C$-Nmr Spectrum in $CDCl_3$, Ppm Downfield from Tetramethylsilane

| | | Hydroxy Acid, Compound IV |
|---|---|---|
| 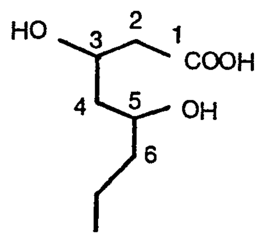 | $C_1$ | 175.0 |
| | $C_2$, $C_4$ | 42.2, 41.7 |
| | $C_3$ | 68.8 |
| | $C_5$ | 72.5 |
| | $C_6$ | 35.0 |

The spectrum of the remainder of the molecule is only slightly changed by the ring closure.

Example 25

*Ethyl Ester of Compound IV*

Employing the procedure substantially as described in Example 19, but substituting for the 1,24 mmol of Compound I used therein, an equimolecular amount of Compound II from Example 20, there is obtained the ethyl ester of Compound IV.

Similarly prepared are the methyl, propyl, butyl, isobutyl, t-butyl, amyl, isoamyl, 2-dimethylaminoethyl, benzyl, phenethyl, and 2-acetamidoethyl esters.

Example 26

A. In Vitro Inhibition of HMG Coenzyme A Reductase

The method of Beg et al., *FEBS Letters, 80,* 123 (1977) was modified slightly by incubating the enzyme with the inhibitor for five minutes before initiating the reaction with the substrate. With potent inhibitors such as the novel compounds of this invention, the standard procedure of merely adding enzyme to the inhibitor-substrate mixture gave non linear kinetics.

Using the modified procedure, the sodium salt of Compound IV gives an $IC_{50}$ in inhibiting HMG-CoA Reductase of $2.7 \times 10^{-9}M$ as compared with $5.4 \times 10^{-9}M$ for ML236B.

B. In Vivo Inhibition of Cholesterol Synthesis (Compound II)

Groups of male Holtzman rats were dosed with either 5% emulphor in saline or test compound in emulphor by stomach tube. After one hour 80 $\mu$Ci of $^{14}C$ acetate/kg was given IP. The rats were bled 50 minutes later and $^{14}C$ cholesterol was determined as a measure of sterol synthesis:

| Dose, mg/kg. | % Inhibition |
|---|---|
| 0.15 | 38 |
| 0.6 | 51 |
| 1.2 | 70 |

Example 27

*Comparison of I, II and ML—236B as Inhibitors of Sterol Synthesis in Cell Culture*

The procedure of A. W. Alberts et al., *J. Biol. Chem. 249:* 5241 (1974) measuring the quantity of $^{14}C$ sterol biosynthesis frofdm $^{14}C$ acetic acid in mouse L—M cells in culture was employed with modification. The test compound in 10 $\mu$l DMSO was added to the monolayer cultures with 5 $\mu$Ci of $^{14}C$ acetate. After a 3 hour incubation, the cells were saponified and the $^{14}C$ sterol extracted and isolated by thin layer chromatography on silica gel using petroleum ether-diethyl ether-acetic acid (75:25:1). The region on the plate containing $^{14}C$ sterol was identified by staining with $I_2$ and the $^{14}C$ content determined by liquid scintillation counting.

Using the modified procedure Compound II gives $IC_{50}$ in inhibiting HMG-CoA reductase of 17 nM as compared to 22 nM for Compound I and 46 nM for ML—236B.

**Claims for the Contracting States: BE CH DE FR GB IT LI LU NL SE**

1. A compound selected from

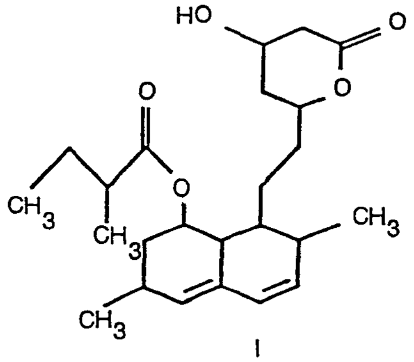
I

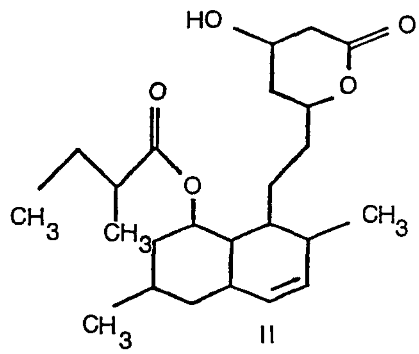
II

2. A compound selected from:

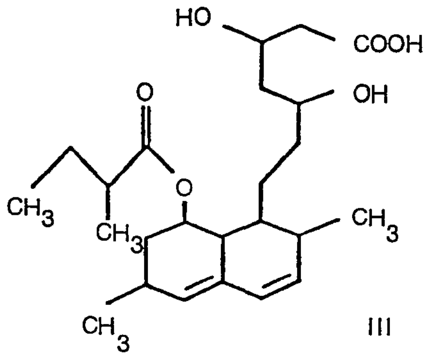
III

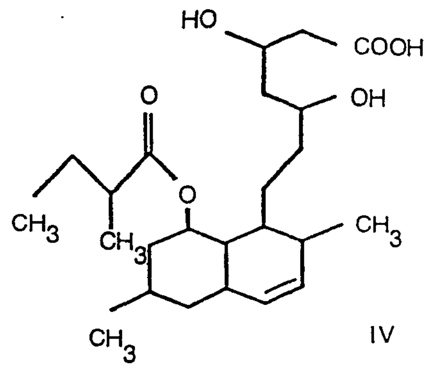
IV

or a pharmaceutically acceptable salt or a lower alkyl ester or a substituted lower alkyl ester wherein the substituent is phenyl, dimethylamino, or acetylamino.

3. The pharmaceutically acceptable salts of the compounds of Claim 2, wherein the cation is derived from ammonia, ethylenediamine, N-methylglucamine, lysine, arginine or ornithine.

4. The ammonium salt of compound III or IV of Claim 2.

5. The lower alkyl esters and substituted lower alkyl esters of the compound of Claim 2.

6. The ethyl ester of Compound III or IV of Claim 2.

7. A process of producing the compounds of structural formulae:

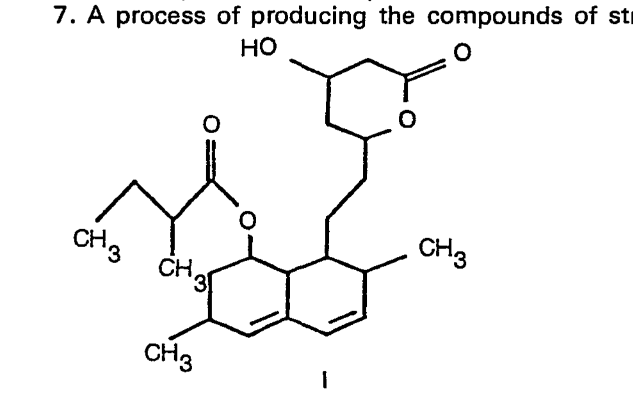
I

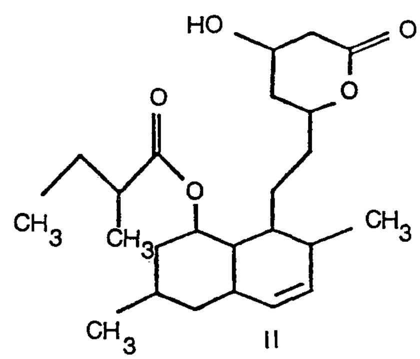
II

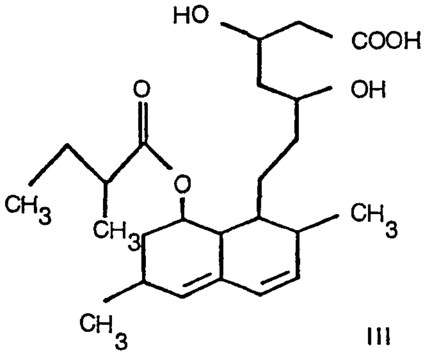
III

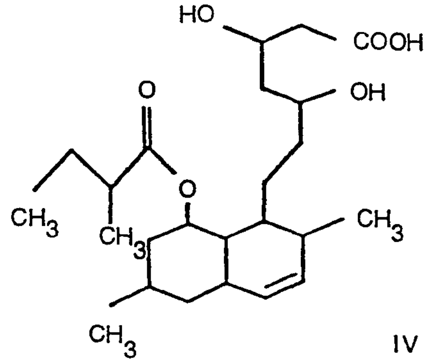
IV

which comprises fermenting a nutrient medium with a microorganism of the species *Aspergillus terreus* and isolating the products.

8. The process of claim 7 in which the microorganism is one deposited in the American Type Culture Collection with Accession number 20541 or 20542.

9. The process of claim 7 in which the isolation comprises extraction of the fermentation mixture with a solvent followed by chromatography.

10. A pharmaceutical composition comprising a pharmaceutical carrier and an effective amount of a compound of any one of Claims 1 to 6.

**Claims for the Contracting State: AT**

1. A process of producing the compounds of structural formulae:

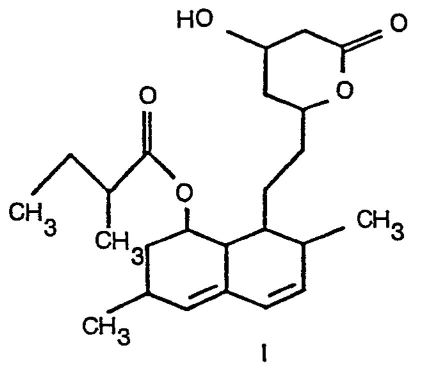

I

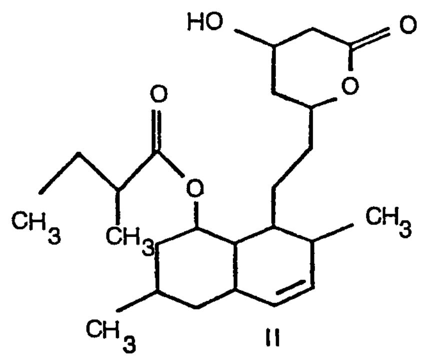

II

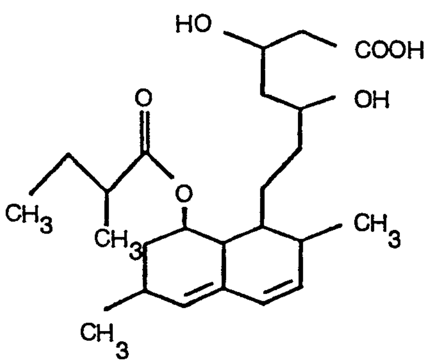

III

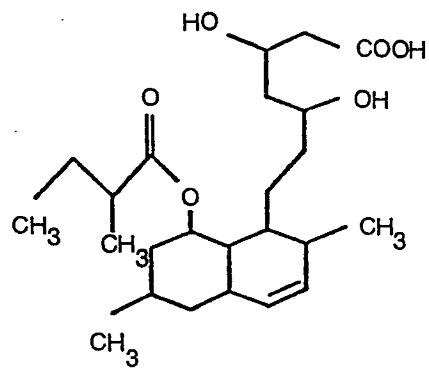

IV

or a pharmaceutically acceptable salt or lower alkyl ester or substituted lower alkyl ester of compounds III or IV wherein the substituent is phenyl, dimethylamino, or acetylamino, which comprises fermenting a nutrient medium with a microorganism of the species *Aspergillus terreus* and isolating the products and, if desired, hydrolyzing compound I or II with a base and, if desired, carefully acidifying the resulting salt to compounds III or IV or, if desired, treating compounds I or II with a lower alkanol or lower alkanol substituted with phenyl, dimethylamino, or acetylamino.

2. The process of producing the compounds of structural formulae:

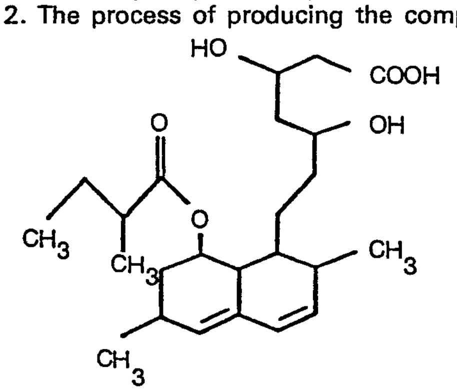

III

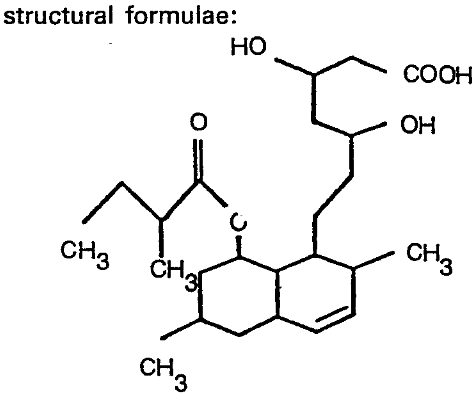

IV

or a pharmaceutically acceptable salt or lower alkyl ester or substituted lower alkyl ester of compounds III or IV wherein the substituent is phenyl, dimethylamino, or acetylamino, which comprises fermenting

a nutrient medium with a microorganism of the species *Aspergillus terreus* and isolating the products and hydrolyzing compounds I or II with a base and, if desired, carefully acidifying the resulting salt to compounds III or IV or treating compounds I or II with a lower alkanol or lower alkanol substituted with phenyl, dimethylamino, or acetylamino.

3. The process of Claims 1 or 2 in which the microorganism is one deposited in the American Type Culture Collection with Accession number 20541 or 20542.

4. The process of Claims 1 or 2 in which the isolation comprises extraction of the fermentation mixture with a solvent followed by chromatography.

5. The process of one of Claims 2 to 4, wherein the base is ammonia, ethylenediamine, N-methylglucamine, lysine, arginine or ornithine.

6. The process of Claims 5, wherein the base is ammonia.

7. The process of one of Claims 2 to 4, wherein the alkanol is ethanol.

**Revendications pour les Etats contractants: BE CH DE FR GB IT LI LU NL SE**

1. Composé choisi entre

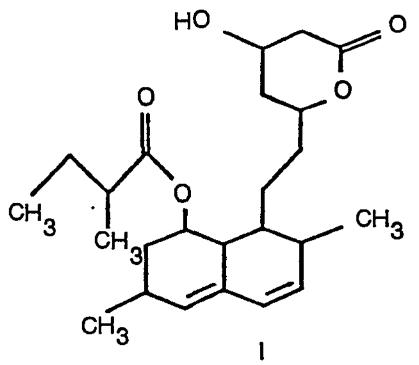

I

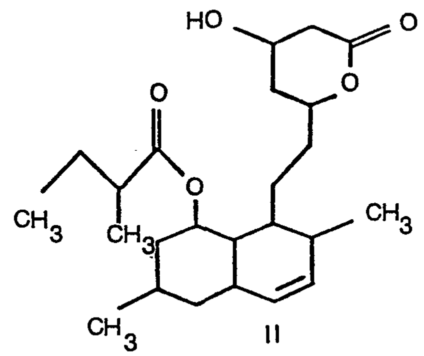

II

2. Composé choisi entre

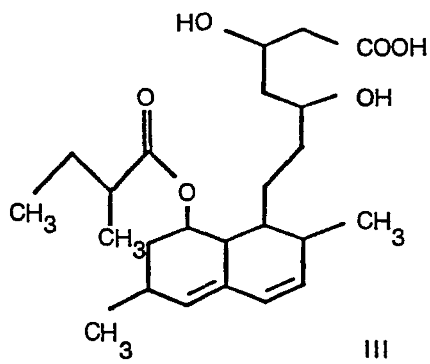

III

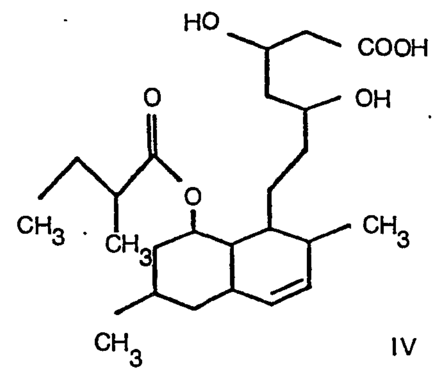

IV

ou un sel pharmaceutiquement acceptable ou un alcoyle inférieur-ester ou un alcoyle inférieur-substitué-ester où le substituant est un phényle, un diméthylamino ou un acétylamino.

3. Sels pharmaceutiquement acceptables des composés de la revendication 2 où le cation provient de l'ammoniac, de l'éthylènediamine, de la N-méthylglucamine, de la lysine, de l'arginine ou de l'ornithine.

4. Sel d'ammonium du composé III ou IV de la revendication 2.

5. Les alcoyle inférieur-esters et alcoyle inférieur-esters substitués des composés de la revendication 2.

6. L'ester éthylique du composé III ou IV de la revendication 2.

7. Procédé de production des composés de formules développées:

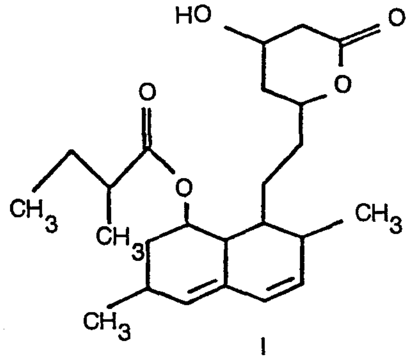

I

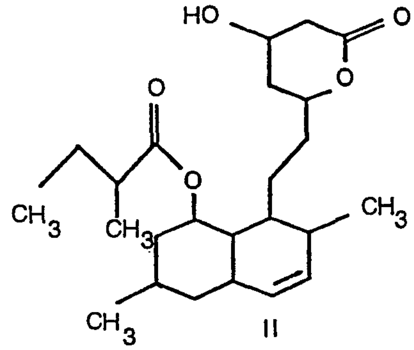

II

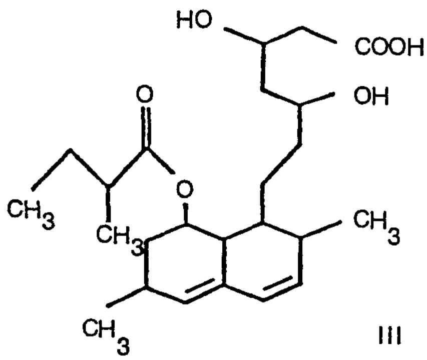

III

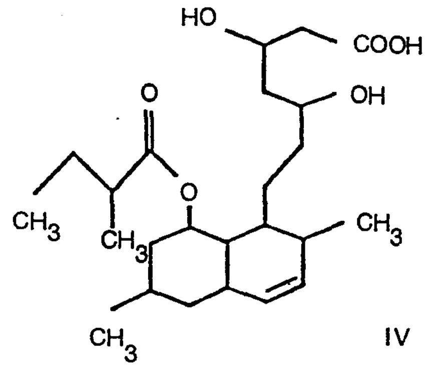

IV

dans lequel on fait fermenter un milieu nutritif avec un microorganisme de l'espèce *Aspergillus terreus* et on isole les produits.

8. Procédé de la revendication 7 où le microorganisme est un microorganisme déposé à l'American Type Culture Collection sous le n° 20541 ou 20542.

9. Procédé de la revendication 7 où l'isolement comprend l'extraction du mélange de fermentation avec un solvant, suivie par une chromatographie.

10. Composition pharmaceutique comprenant un support pharmaceutique et une quantité efficace d'un composé de l'une quelconque des revendications 1 à 6.

**Revendications pour l'Etat contractant: AT**

1. Procédé de production des composés de formules développées

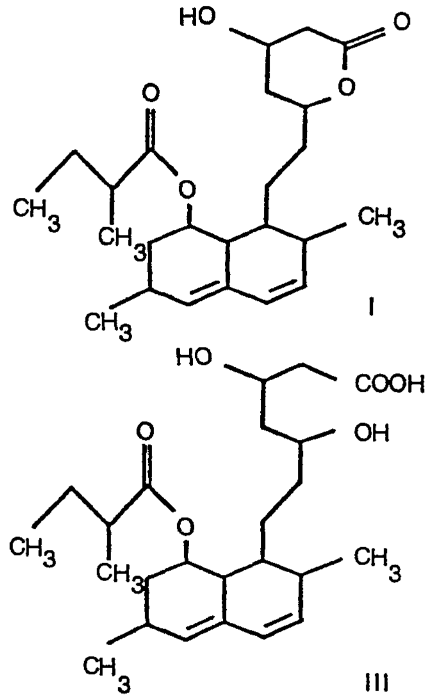

I

III

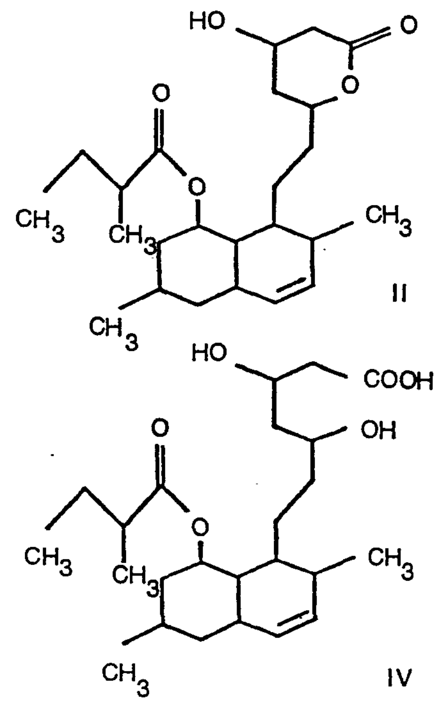

II

IV

ou d'un sel pharmaceutiquement acceptable ou d'un alcoyle inférieur-ester ou alcoyle inférieur substitué-ester des composés III ou IV où le substituant est unphényle, un diméthylamino ou un acétylamino, dans lequel on fait fermenter un milieu nutritif avec un microorganisme de l'espèce *Aspergillus terreus* et on isole les produits et, si on le désire, on hydrolyse le composé I ou II avec une base et, si le on le désire, on acidifie avec soin le sel résultant en composés III ou IV ou, si on le désire, on traite les composés I ou II avec un alcanol inférieur ou un alcanol inférieur substitué par un phényle, un diméthylamino ou un acétylamino.

2. Procédé de production des composés de formules développées:

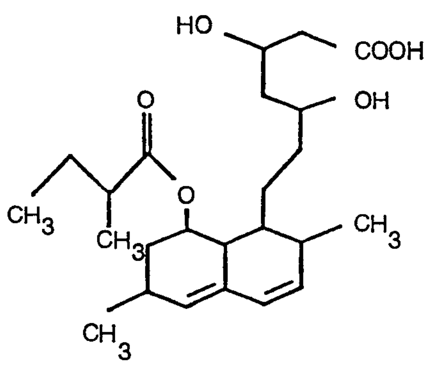

III

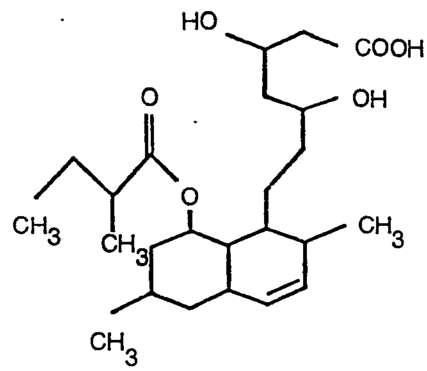

IV

ou d'un sel pharmaceutiquement acceptable ou d'un alcoyle inférieur-ester ou alcoyle inférieur-ester substitué de composés III ou IV où le substituant est un phényle, un diméthylamino ou un acétylamino, dans lequel on fait fermenter un milieu nutritif avec unmicroorganisme de l'espèce *Aspergillus terreus* et on isole les produits et on hydrolyse les composés I ou II avec une base et, si on le désire, on acidifie avec soin le sel résultant en composés III ou IV ou on traite les composés I ou II avec un alcanol inférieur ou un alcanol inférieur substitué par un phényle, un diméthylamino ou un acétylamino.

3. Procédé des revendications 1 ou 2 où le microorganisme est un microorganisme déposé à l'American Type Culture Collection sous le n° 20541 ou 20542.

4. Procédé de la revendication 1 ou 2 où l'isolement comprend l'extraction du mélange de fermentation avec un solvent suivie par une chromatographie.

5. Procédé de l'une des revendications 2 à 4, où la base est l'ammoniac, l'éthylènediamine, la N-méthylglucamine, la lysien, l'arginine ou l'ornithine.

6. Procédé de la revendication 5 où la base est l'ammoniac.

7. Procédé de l'une des revendications 2 à 4, où l'alcanol est l'éthanol.

**Patentansprüche für die Vertragsstaaten: BE CH DE FR GB IT LI LU NL SE**

1. Eine Verbindung, ausgewählt aus

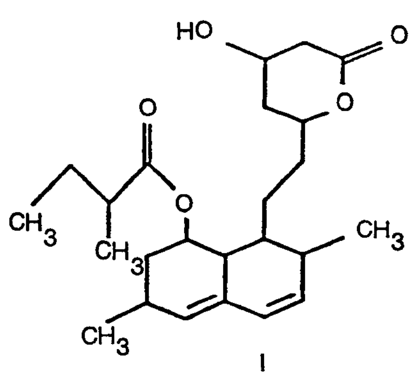

I

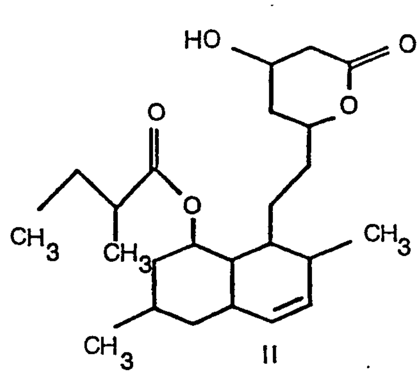

II

2. Eine Verbindung, ausgewählt aus

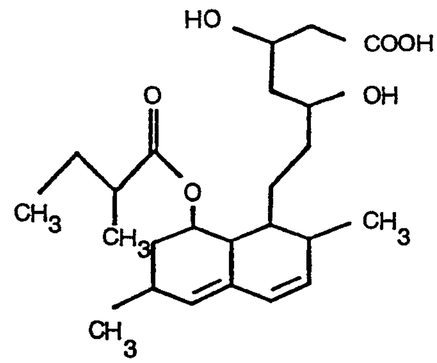

III

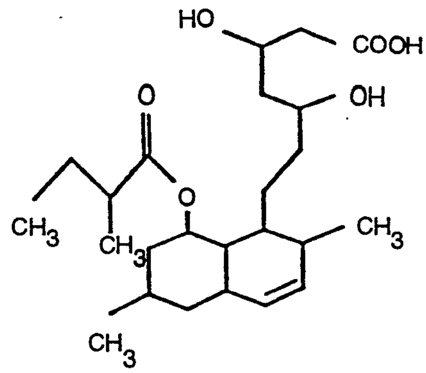

IV

oder ein pharmazeutisch annehmbares Salz oder ein Niedrigalkylester oder ein substituierter Niedrigalkylester davon, worin der Substituent Phenyl, Dimethylamino oder Acetylamino ist.

3. Die pharmazeutisch annehmbaren Salze der Verbindungen des Anspruchs 2, worin das Kation von Ammoniak, Ethylendiamin, N-Methylglucamin, Lysin, Arginin oder Ornithin abgeleitet ist.

4. Das Ammoniumsalz der Verbindung III oder IV des Anspruchs 2.

5. Die Niedrigalkylester und substituierten Niedrigalkylester der Verbindungen des Anspruchs 2.

6. Der Ethylester der Verbindung III oder IV des Anspruchs 2.

7. Ein Verfahren zur Herstellung der Verbindungen der Strukturformeln

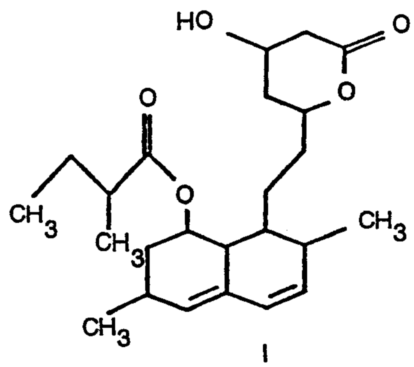

I

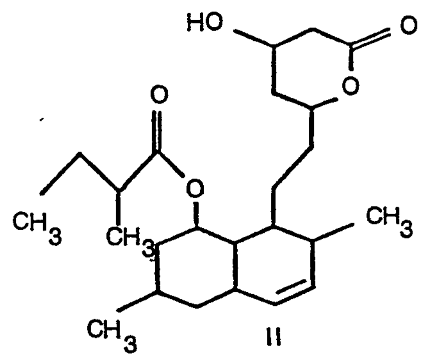

II

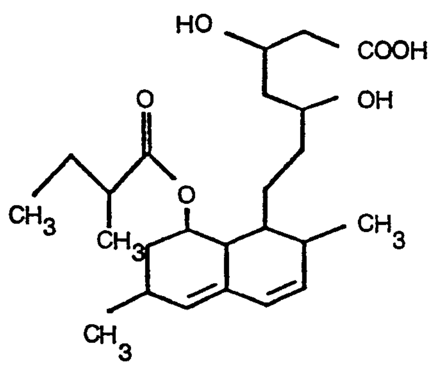

III

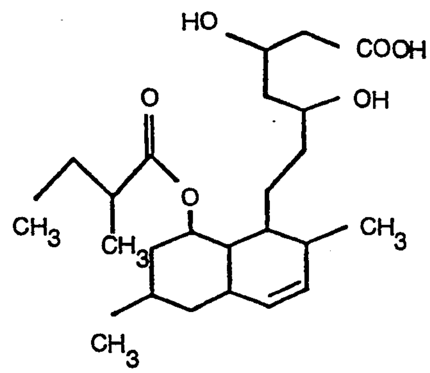

IV

umfassend das Fermentieren eines Nährmediums mit einem Mikroorganismus der Spezies Aspergillus terreus und das Isolieren der Produkte.

8. Das Verfahren nach Anspruch 7, worin der Mikroorganismus ein solcher ist, der in der American Type Culture Collection unter der Accession Nummer 20541 oder 20542 hinterlegt ist.

9. Das Verfahren nach Anspruch 7, worin die Isolierung das Extrahieren des Fermentationsgemisches mit einem Lösungsmittel und anschließendes Chromatographieren umfaßt.

10. Eine pharmazeutische Zusammensetzung, umfassend einen pharmazeutischen Träger und eine wirksame Menge einer Verbindung nach einem der Ansprüche 1 bis 6.

**Patentansprüche für den Vertragsstaat: AT**

1. Ein Verfahren zur Herstellung der Verbindungen der Strukturformeln

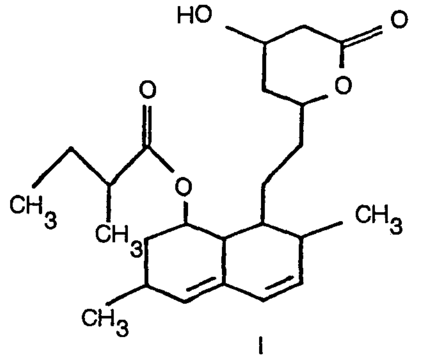

I

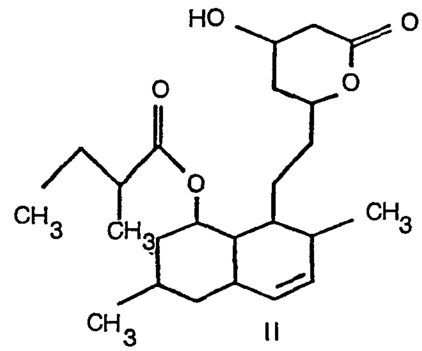

II

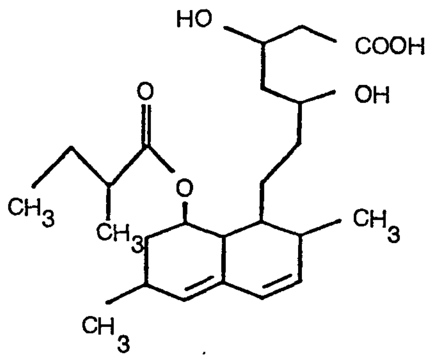

III

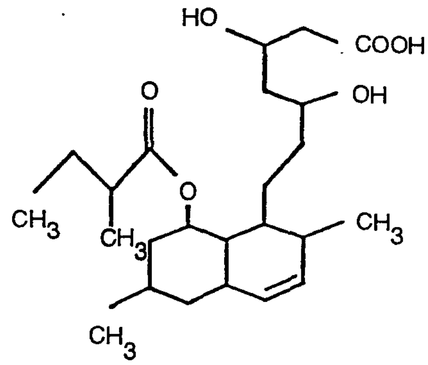

IV

oder eines pharmazeutisch annehmbaren Salzes oder Niedrigalkylsters oder substituierten Niedrigalkylesters der Verbindungen III oder IV, worin der Substituent Phenyl, Dimethylamino oder Acetylamino ist, umfassend das Fermentieren eines Nährmediums mit einem Mikroorganismus der Spezies Aspergillus terreus und das Isolieren der Produkte und, gewünschtenfalls, das Hydrolysieren der Verbindung I oder II mit einer Base und, gewünschtenfalls, das vorsichtige Ansäuern des erhaltenen Salzes zur Verbindung III oder IV oder, gewünschtenfalls, die Behandlung der Verbindung I oder II mit einem niedrigen Alkanol oder durch Phenyl, Dimethylamino oder Acetylamino substituierten niedrigen Alkanol.

2. Das Verfahren zur Herstellung der Verbindungen der Strukturformeln

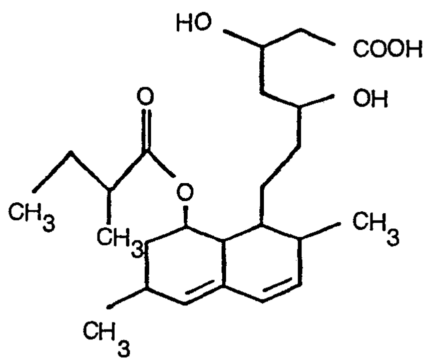

III

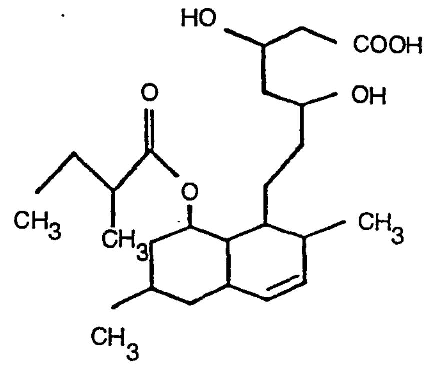

IV

oder eines pharmazeutisch annehmbaren Salzes oder Niedrigalkylesters oder substituierten Niedrigalkylesters der Verbindungen III oder IV, worin der Substituent Phenyl, Dimethylamino oder Acetylamino ist, umfassend das Fermentieren eines Nährmediums mit einem Mikroorganismus der Spezies Aspergillus terreus und das Isolieren der Produkte und das Hydrolysieren der Verbindung I oder II mit einer Base und, gewünschtenfalls, das vorsichtige Ansäuern des erhaltenen Salzes zu Verbindung III oder IV oder die Behandlung der Verbindung I oder II mit einem niedrigen Alkanol oder durch Phenyl, Dimethylamino oder Acetylamino substituierten niedrigen Alkanol.

3. Das Verfahren nach Anspruch 1 oder 2, worin der Mikroorganismus ein solcher ist, der in der American Type Culture Collection unter der Accession Nummer 20541 oder 20542 hinterlegt ist.

4. Das Verfahren nach Anspruch 1 oder 2, worin die Isolierung das Extrahieren des Fermentationsgemisches mit einem Lösungsmittel und anschließendes Chromatographieren umfaßt.

5. Das Verfahren nach einem der Ansprüche 2 bis 4, worin die Base Ammoniak, Ethylendiamin, N-Methylglucamin, Lysin, Arginin oder Ornithin ist.

6. Das Verfahren nach Anspruch 5, worin die Base Ammoniak ist.

7. Das Verfahren nach einem der Ansprüche 2 bis 4, worin das Alkanol Ethanol ist.

FIG. 1

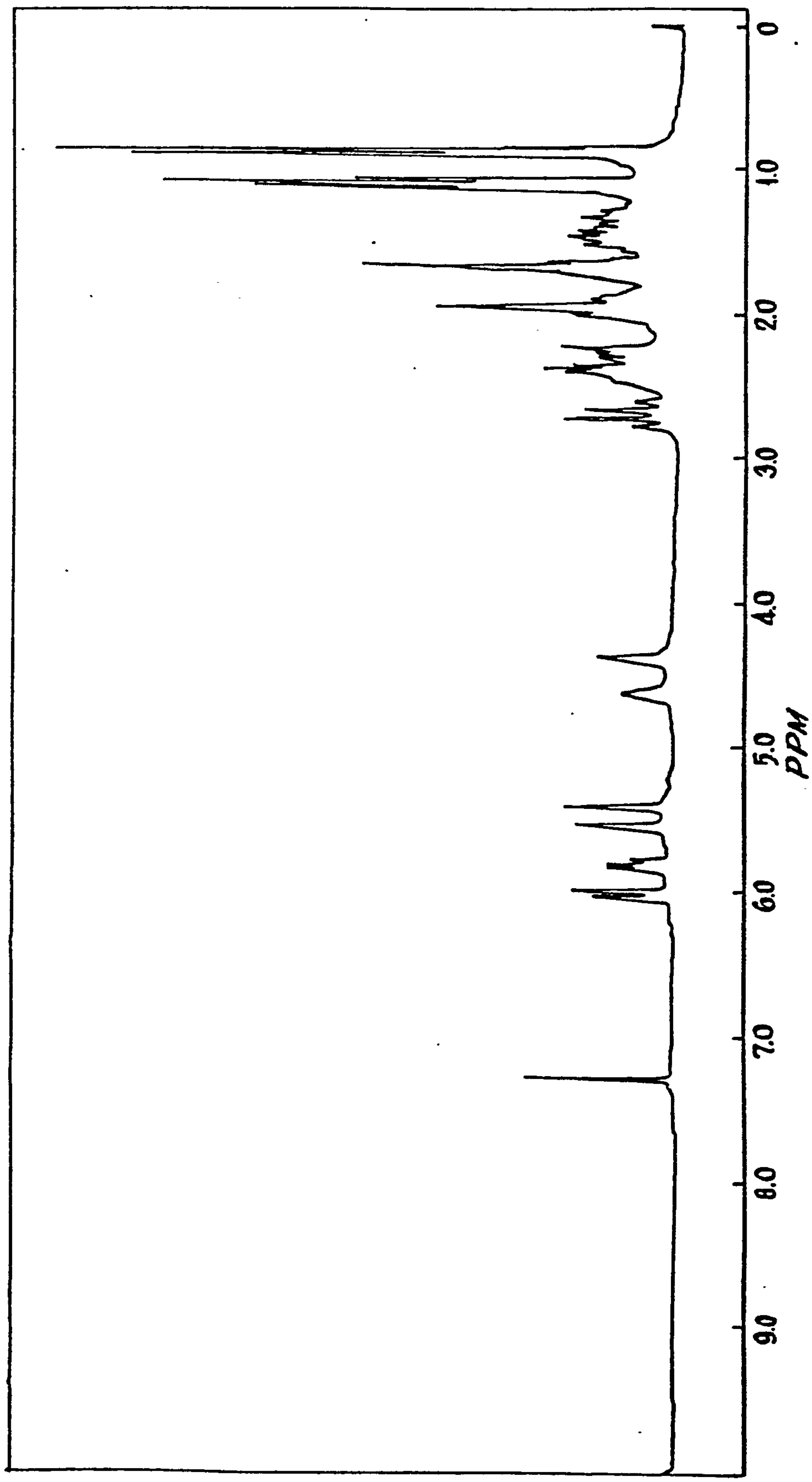

FIG. 2

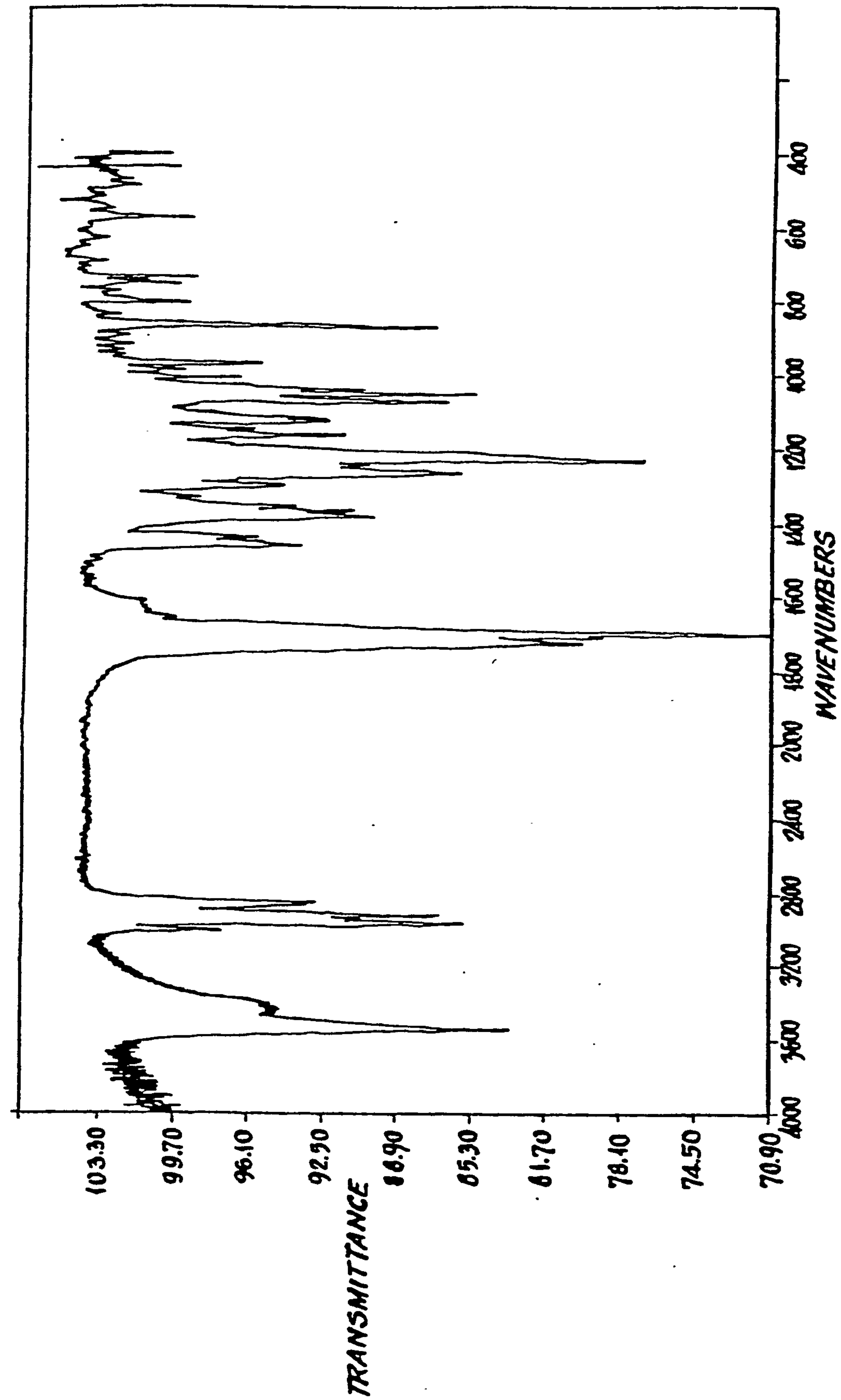
TRANSMITTANCE
103.30
99.70
96.10
92.50
88.90
85.30
81.70
78.10
74.50
70.90
WAVENUMBERS
4000
3600
3200
2800
2400
2000
1600
1200
800
400

FIG. 3

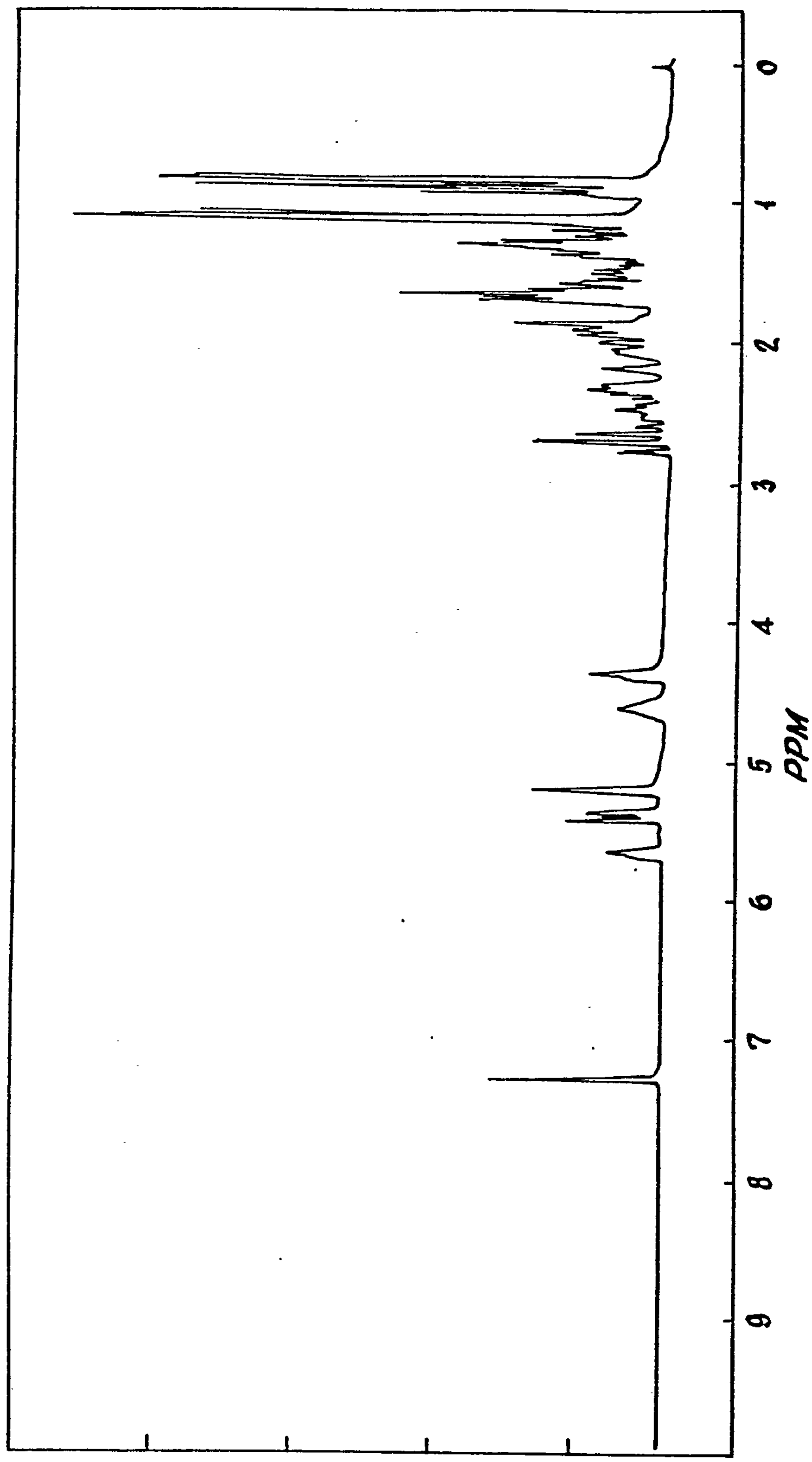

FIG. 4

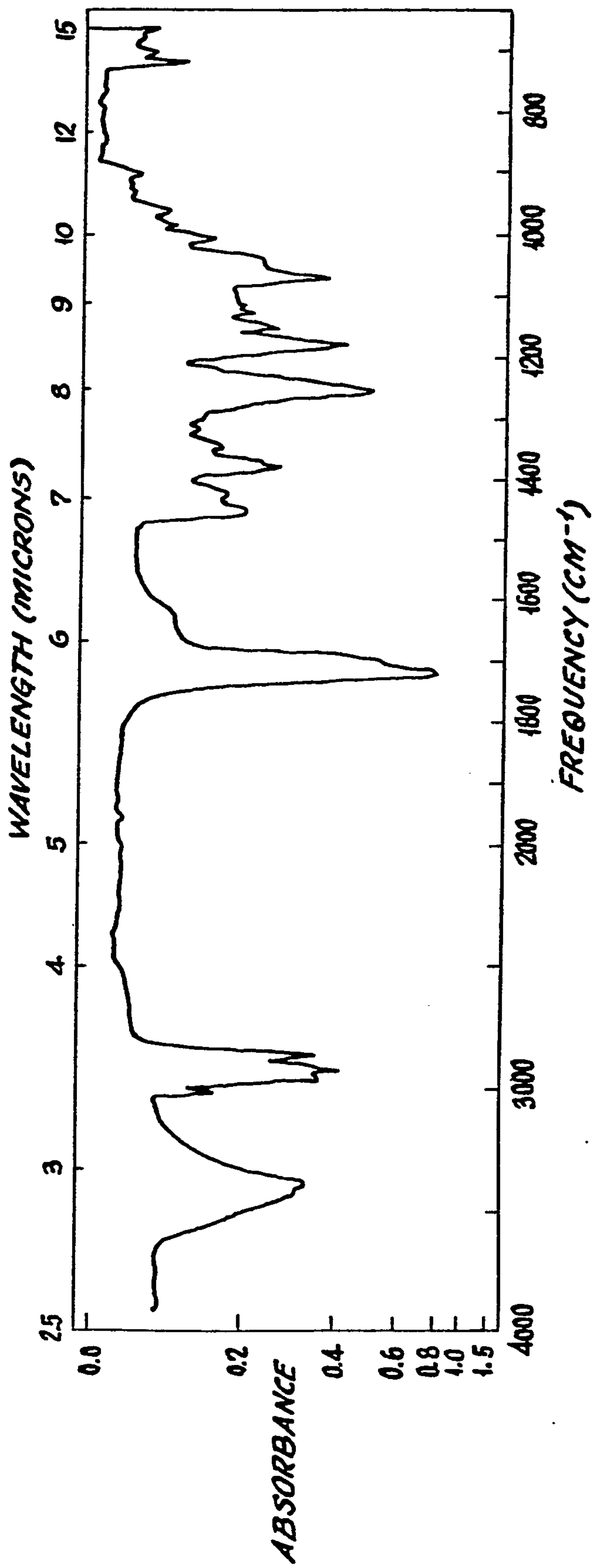
WAVELENGTH (MICRONS)
2.5
3
4
5
6
7
8
9
10
12
15
FREQUENCY (CM⁻¹)
4000
3000
2000
1800
1600
1400
1200
1000
800
ABSORBANCE
0.0
0.2
0.4
0.6
0.8
1.0
1.5